# EUROPEAN PATENT SPECIFICATION

(11) **EP 2 999 486 B1**
(45) Date of publication and mention of the grant of the patent: **01.07.2020**
(21) Application number: 14800309.8
(22) Date of filing: 20.05.2014
(51) Int. Cl.: A61K 47/02, A61K 47/36, A61K 47/42, A61P 9/10

(54) **METAL-COATED SCAFFOLDS FOR TISSUE ENGINEERING**
METALLBESCHICHTETE GERÜSTE FÜR GEWEBE-ENGINEERING
ÉCHAFAUDAGES À REVÊTEMENT MÉTALLIQUE POUR INGÉNIERIE TISSULAIRE

(30) Priority: 20.05.2013 US 201361825124 P
(43) Date of publication of application: 30.03.2016
(73) Proprietor: Ramot at Tel-Aviv University Ltd., 6139201 Tel-Aviv (IL)
(72) Inventor: DVIR, Tal, 6139201 Tel-Aviv (IL); SHEVACH, Michal, 6291751 Tel-Aviv (IL); MAOZ, Ben M., 4664017 Herzlia (IL)
(74) Representative: Luppi, Emanuele
(86) International application number: PCT/IL2014/050445
(87) International publication number: WO 2014/188420

(56) References cited:
- WO-A1-2013/109642
- US-A1- 2009 163 990
- US-A1- 2010 106 233
- US-A1- 2010 106 233
- US-A1- 2010 255 447
- COHEN-KARNI, T. ET AL.: 'Nanocomposite gold-silk nanofibers.' NANO LETTERS vol. 12, no. 10, 2012, pages 5403 - 5406, XP055295588
- YOU, J. O. ET AL.: 'Nanoengineering the heart: conductive scaffolds enhance connexin 43 expression.' NANO LETTERS vol. 11, no. 9, 2011, pages 3643 - 3648, XP055204904
- Jin-Oh You ET AL: "Nanoengineering the Heart: Conductive Scaffolds Enhance Connexin 43 Expression", Nano Letters, vol. 11, no. 9, 14 September 2011 (2011-09-14), pages 3643-3648, XP055204904, ISSN: 1530-6984, DOI: 10.1021/nl201514a
- Tal Dvir ET AL: "Nanowired three-dimensional cardiac patches", NATURE NANOTECHNOLOGY, vol. 6, no. 11, 25 September 2011 (2011-09-25), pages 720-725, XP55526382, GB ISSN: 1748-3387, DOI: 10.1038/nnano.2011.160

## Description

### FIELD AND BACKGROUND OF THE INVENTION

The present invention, in some embodiments thereof, relates to scaffolds coated with metal nanoparticles.

Transplantation of engineered cardiac patches is a promising strategy for improving the function of diseased hearts. In this approach cardiac cells are seeded within 3-dimensional (3D) biomaterial scaffolds to induce quick assembly into a functioning cardiac tissue. Then, these cardiac patches are transplanted to replace the injured tissue and regain function. In recent years engineering strategies were focused on inducing cardiac specific morphogenesis, maintaining cell viability, and promoting proper function of the engineered tissue. Thus, bioreactors providing enhanced mass transfer increased cell survival, and electrical and mechanical signals were able to promote the formation of typical cardiac ultrastructural morphology. In an attempt to induce cell-matrix interactions within the engineered 3D microenvironments, the surface of scaffolds was chemically modified with ECM proteins or with their functional motifs. Such modification has led to the formation of elongated and aligned cells, resembling the morphology of cell of the natural myocardium. Micro and nanofabrication processes were developed to create 2-dimensional (2D) substrates with various topographies for inducing cell adhesion, and for controlling cardiac cell assembly and morphogenesis.

Dvir et al., 2011, Nature nanotechnology 6, 720-725, teaches embedding of gold nanowires within the pore walls of macroporous alginate scaffolds to increase the spatial and overall conductivity of the matrix. When cardiac cells were cultured in these nanowired scaffolds they exhibited increased expression of contractile and electrical coupling proteins.

You et al, 2011, Nano letters 11, 3643-3648 teaches cardiomyocytes cultured in hybrid hydrogel scaffolds based on spherical gold NPs, homogeneously distributed throughout a polymeric gel. The cells exhibited increased expression of connexin 43, a protein located between cardiac cells, responsible for electrical signal transfer.

Prabhakaran et al, 2011 Journal of nanoscience and nanotechnology 11, 3039-3057 teaches electrospinning to fabricate fibrous scaffolds from various polymers.

Whelove et al., 2011, J Biomed Mater Res Part B 2011:99B:142-149 teaches nanoparticles conjugated to the surface of Polyethylene terephthalate scaffolds.

Karni et al, Nano letters, 2012, 12(10), 5403-5406, teaches electrospinning of silk fibers together with gold nanoparticles for culturing mesenchymal stem cells.

Shan-hui Hsu Biomacromolecules 2008, 9, 241-248 teaches polyurethane gold composite films.

Prabhakaran et al, 2012, Biopolymers Volume 97, 7, pages 529-538 teaches electrospun POC and PLCL scaffolds for cardiac engineering. To view under a scanning electron microscope, the scaffolds were sprayed with gold.

Fleischer et al Biomaterials, 2013, 34(34), 8599-8606 teaches coiled fibers for cardiac tissue engineering.

U.S. Patent Application 20100106233 teaches decellularized tissue covered with functionalized gold nanoparticles for soft tissue repair.

### SUMMARY OF THE INVENTION

The scope of the invention is defined by the claims: Any references in the description to methods of treatments refer to the composition for use in a method for treatment of the human or animal body by therapy.

According to an embodiment of the present invention there is provided a composition of matter according to claims 1-7.

According to an other embodiment of the present invention there is provided a method of generating a scaffold seeded with cells according to claims 8-12.

According to a further embodiment of the present invention there is provided a composition of matter of any of claims 1-7 for use in treating a disease or disorder associated with a decrease in activity or amount of electrically excitable cells.

According to some embodiments of the invention, the metal nanoparticles comprise gold nanoparticles.

According to some embodiments of the invention, the scaffold comprises fibers. The coating is between 2-20 nm in thickness, preferably between 4-14 nm in thickness.

According to some embodiments of the invention, the fibers comprise electro spun fibers.

According to some embodiments of the invention, the fibers comprise a non-biodegradable polymer.

According to some embodiments of the invention, the scaffold comprises decellularized extracellular matrix (ECM).

According to some embodiments of the invention, the fibers comprise a biodegradable polymer.

According to some embodiments of the invention, the biodegradable polymer is selected from the group consisting of polycaprolactone (PCL), polylactic acid (PLA), polyglycolic acid (PGA), and poly(Lactide-co-Glycolide) (PLGA).

According to some embodiments of the invention, the composition of matter further comprises an adhesive agent.

According to some embodiments of the invention, the adhesive agent is selected from the group consisting of gelatin, fibrin, fibronectin, collagen and RGD.

According to some embodiments of the invention, the composition of matter further comprises at least one agent for promoting cell adhesion, colonization, proliferation, differentiation, extravasation and/or migration.

According to some embodiments of the invention, the proliferation promoting agent comprises a growth factor.

According to some embodiments of the invention, the growth factor comprises VEGF.

According to some embodiments of the invention, the scaffold further comprises electrically excitable cells seeded on the coating.

According to some embodiments of the invention, the electrically excitable cells are selected from the group consisting of muscle cells, glandular cells and nerve cells.

According to some embodiments of the invention, the muscle cells comprise cardiac muscle cells.

According to some embodiments of the invention, the metal nanoparticles are non-homogeneously distributed in the coating.

According to some embodiments of the invention, the metal nanoparticles are attached to an antibody.

According to some embodiments of the invention, the antibody recognizes a cell surface marker.

According to some embodiments of the invention, the antibody recognizes a cell-secreted factor.

According to some embodiments of the invention, the nanoparticles consist of gold.

According to some embodiments of the invention, the metal comprises gold.

According to some embodiments of the invention, the method further comprises electrospinning fibers of the scaffold prior to step (a).

According to some embodiments of the invention, the method further comprises decellularizing a tissue to generate fibers of the scaffold prior to step (a).

According to some embodiments of the invention, the disease is a cardiac disease.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIGs. 1A-D. Schematic overview of the study. A. Fabrication of PCL/gelatin fiber scaffolds by electro spinning. B. Evaporation of gold NPs onto the fibers to create nanocomposite scaffolds. C. and D. Cardiac cells are seeded in the nanocomposite scaffolds for engineering a functional cardiac tissue.
FIGs. 2A-F. Gold NP fiber scaffolds. A. Macroscopic pictures of the scaffolds with varying nominal thickness of gold. Upper left and clockwise: no gold, 2 nm, 4 nm , and 14 nm. B. eSEM image of the 4 nm scaffold. Gold NPs were evaporated on the lower left part (bright part) while the upper right part is without gold. As shown, the morphology of the fibers was not affected due to the introduction of gold. Bar= 20 µm. C-F. Fiber morphology by light microscope. C. No gold. D. 2 nm. E. 4 nm. F. 14 nm. Bar= 5 µm.
FIGs 3A-C. eSEM micrographs of the scaffolds. Upper and lower panels are low and high magnifications, respectively. A. Scaffolds with nominal thickness of 2 nm. B .Nominal thickness of 4nm. C. Nominal thickness of 14nm. Bars- upper panels= 2 µm, lower panels= 300 nm.
FIGs. 4A-E. NPs on the fibers. A. Illustration of fiber cross section. B, cross section TEM of the fibers coated with gold NPs. Bar= 100 nm. C. Illustration of a single fiber covered with gold NPs. D. Topography of gold NP fibers by AFM. E. Topography of gold NPs (4 nm) on a single fiber.
FIG. 5A-B. Reflectance measurements. A. A localized surface plasmon resonance (LSPR) peak can be seen in the 2 nm, and 4 nm samples. The 14 nm has a broad peak as expected. B. A Plasmon shift due to the cells located on the gold scaffolds.
FIGs. 6A-D. Cardiac cell organization on the scaffolds. Cardiac sarcomeric actinin immunostaining on day 7. A. Cardiac cells cultured on pristine scaffolds without gold, mostly exhibited a rounded morphology. B Cardiac tissue engineered in 2 nm gold NP scaffolds. C. Cardiac tissue engineered in 4 nm gold NP scaffolds. D. Cardiac tissue engineered in 14 nm gold NP scaffolds. In the 14 nm gold scaffolds the cells exhibited elongated and aligned morphology. Bar = 100 µm (left), 20 µm (right). Actinin- pink, Nuclei- blue.
FIGs. 7A-B. Cell characteristics in the scaffolds. A. Cell aspect ratio. B. The ratio of cardiomyocytes to cardiac fibroblasts in the different scaffolds. * indicates p= 0.003 ** ,indicates p= 0.0004.
FIGs. 8A-D. Engineered cardiac tissue function. A. Contraction amplitude on day 3. B .Contraction rate on day 3. C. Contraction amplitude on day 7. D. Contraction rate on day 7. n ≥ 5 in each group.
FIG. 9. Schematic representation of the method, according to some embodiments of the present invention.
FIGs. 10A-F. Native and synthetic coiled fibers. (A) SEM image of the coiled perimysial fibers in a decellularized heart. (B) ESEM image of electrospun coiled fiber embedded with AuNPs. (C, D) SEM images of coiled fiber scaffolds. (E) ESEM image of the AuNPs embedded on the synthetic fiber. (F) EDX spectrum of AuNPs coiled fibers. Bars: A, B and D- 20 µm, C- 50 µm, E- 250 nm.
FIGs. 11A-C. Topography and mechanical properties by AFM. (A) Topography of a coiled fiber supplemented with AuNPs. (B) Topography of a non-modified coiled fiber. (C) Analysis of Young's modulus by AFM.
FIGs. 12A-G. Cardiac tissue organization and function. (A, B) Cardiac sarcomeric actinin immunostaining on day 7. Actinin - pink, nuclei - blue. (A) Cardiac tissue engineered within pristine scaffolds. (B) Cardiac tissue engineered within AuNPs scaffolds. (C) Cardiomyocyte area on day 3 and 7. (D) Cardiomyocyte aspect ratio on days 3 and 7. (E-G) Engineered tissue function. (E) Contraction rate on day 7. (F) Longitude change of the cell constructs on day 7. (G) Excitation threshold (ET) on day 7. Bars: left and right figures- 50 µm and 20 µm, respectively.
FIGs. 13A-C. Bar graphs illustrating mechanical measurements of the cardiac cells seeded on the scaffolds described herein.
FIG. 14. Schematic illustration of an embodiment of the present invention. Omental tissue is extracted from a patient. The tissue is decellularized. Gold nano particles (NPs) are evaporated on the fibers. Cells are seeded on the composite scaffold to assemble into a functioning tissue *in vivo.* The scaffold is transplanted to the patient.
FIGs. 15A-B. Photographs illustrating Omental tissue prior to (15A) and following (15B) decellularization process.
FIGs. 15C-D. Photographs illustrating omental tissue after lyophilization (Figure 15C). Pristine scaffold and 4 and 10 nm evaporated scaffolds (Figure 15D). Note the change of color with the change in nominal NPs size.
FIGs. 16A-C. ESEM pictures of gold NPs of various sizes on the fibers.
FIG. 17A. EDX measurement show Au element on the fibers.
FIGs. 17B-C. Current-Voltage diagram of pristine, 4 and 10 nm evaporated scaffolds.
FIG. 17D. Resistance of pristine and evaporated scaffolds.
FIGs. 18A-B. ESEM image (Figure 18A) and EDX measurement (Figure 18B) of 10 nm evaporated scaffolds after 6 days in mild shaking in DDW. Note the Au pick on the fiber but not on the underlying matrix.
FIGs. 19A-B. TEM images following scaffold degradation for 5 days in collagenase-containing buffer.
FIG. 19C. Graph illustrating the distribution of nanoparticle diameter after degradation.
FIGs. 20A-C. Cardiac cells cultivated on pristine (A), 4(B) and 10 (C) nm evaporated scaffolds.
FIGs. 21A-C. Contraction amplitude (Figure 21A), excitation threshold (minimal applied voltage for synchronies scaffold contraction with applied frequency; Figure 21B) and electrical signal propagation velocity (Figure 21C) of cardiac cells seeded on pristine, 4 and 10 nm evaporated scaffolds.
FIG. 22. Viability (XTT test) of cardiac fibroblasts on day 3 post seeding on the scaffolds.
FIGs. 23A-B. VEGF relative fluorescence after staining. Figure 23A: pristine scaffold with VEGF. Figure 23B: 10 nm evaporated scaffold with VEGF.

### DESCRIPTION OF SPECIFIC EMBODIMENTS OF THE INVENTION

The present invention, in some embodiments thereof, relates to scaffolds coated with metal nanoparticles. The scaffolds may be used for tissue engineering and more specifically for cardiac tissue engineering.

Treatments for chronic heart failure include medical management with pharmaceutical drugs, diet and exercise, and mechanical assist devices, which are costly and risk failure and infection. The results of all these effort are disappointing with a 75 % five year mortality rate for heart failure victims. Thus, the landscape for cardiac treatment is turning in recent years to transplantation of tissue or cells. However, as of yet, these transplants have only met with a limited success rate.

The modern approach to cardiac tissue engineering is the design and fabrication of porous three dimensional (3D) scaffolds which serve as temporary artificial extracellular matrices, accommodating cells and supporting 3D tissue regeneration.

It is known that the incorporation of different nanoscale structures of gold into biomaterials can improve the engineered cardiac tissue structure and function. However, scaffolds used in these studies did not mimic the fibrous structure of the natural microenvironment, which induces a proper organization of the cells.

The present inventors have devised a new approach for fabricating 3-dimensional (3D) gold nanoparticle (NP)-based scaffolds, for tissue engineering. They evaporated gold nanoparticles (NPs) on the surface of the scaffold, creating nanocomposites with nominal gold thickness between 2-20 nm. The thickness of the evaporated gold film on the scaffolds allowed for control of island size.

Whilst reducing the present invention to practice, the present inventors generated different types of scaffolds (both electrospun and decellularized extracellular matrix (ECM) upon which they added an external layer of gold nanoparticles.

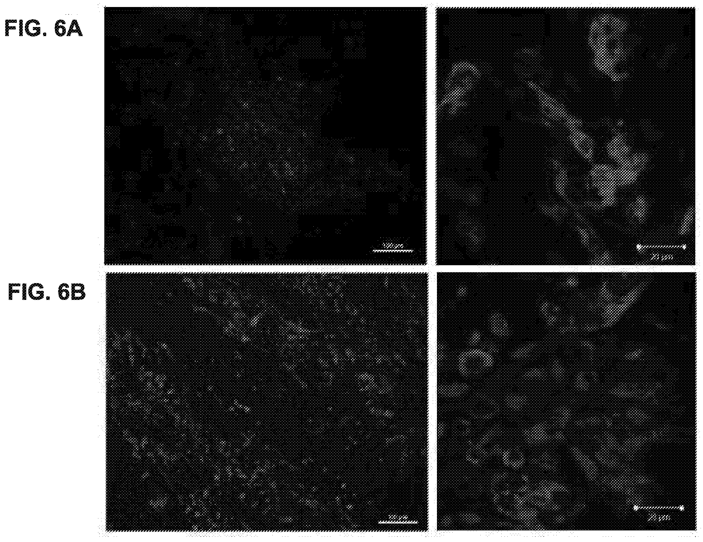

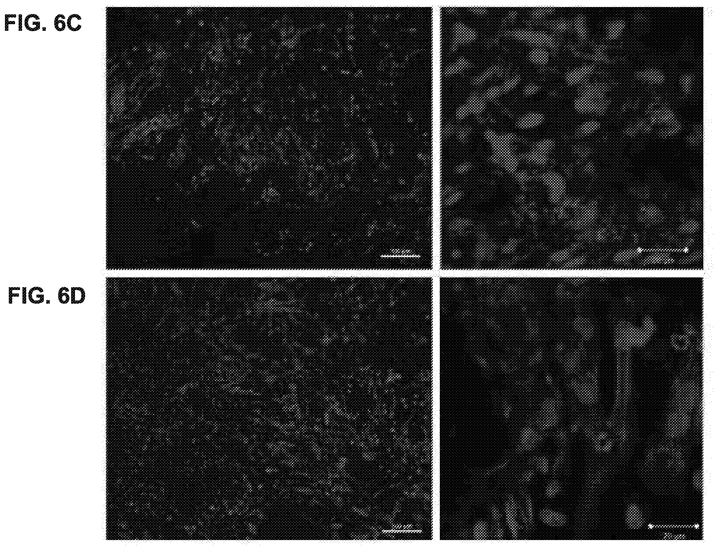

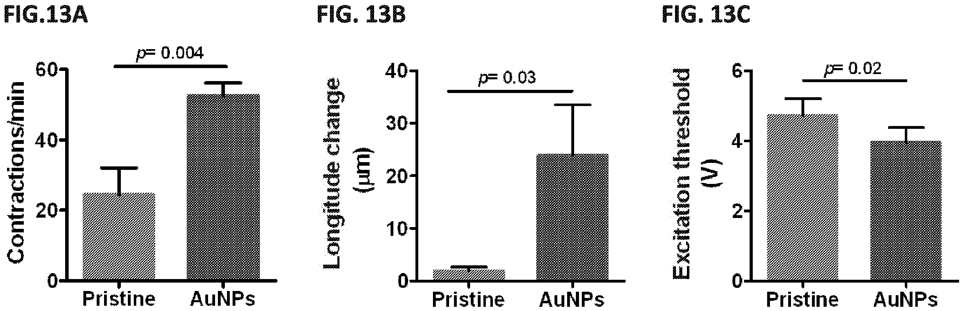

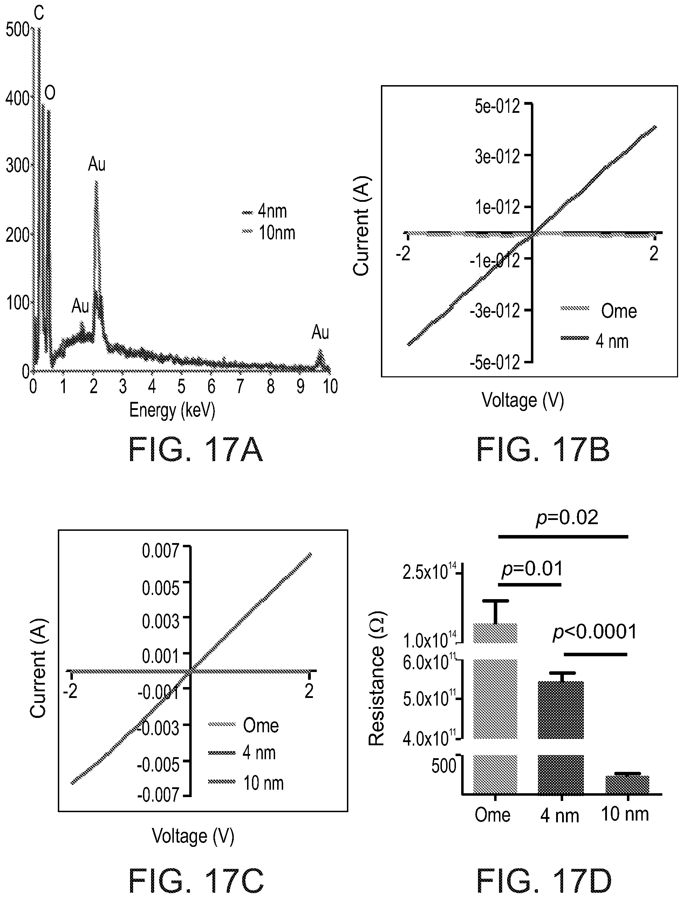

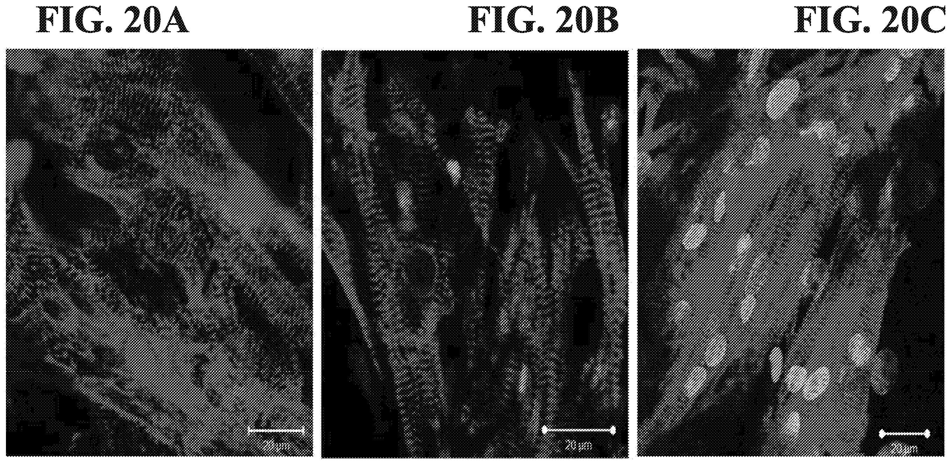

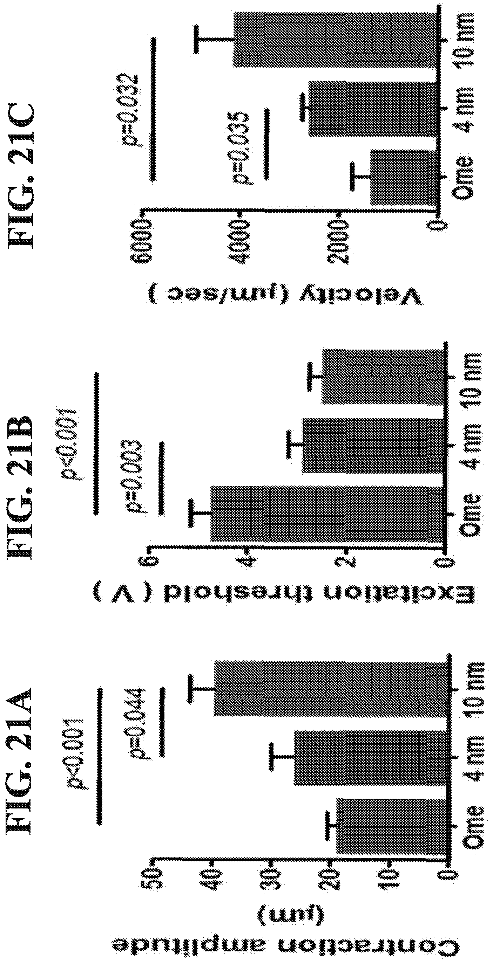

As demonstrated in the Examples section herein below, compared to pristine scaffolds, cardiac cells seeded on the nano-gold scaffolds assembled into more elongated and aligned tissues. This has been shown for electrospun scaffolds (Figures 6A-D; 7A-B, 10A-F, 11A-C and 12A-G) and scaffolds prepared from decellularized tissue (Figures 13A-C, 17B-C, 20A-C and 21A-C). The gold NPs on the fibers were able to maintain the ratio of cardiomyocytes to fibroblasts in the culture, to encourage the growth of cardiomyocytes with significantly higher aspect ratio, and promote massive cardiac sarcomeric actinin expression. Finally, engineering cardiac tissues within gold NP-based scaffolds exhibited significantly higher contraction amplitudes and rates, as compared to scaffolds without gold.

The present inventors propose that cardiac tissues engineered within and upon these gold NP scaffolds can be used to improve the function of the infarcted heart.

Thus, according to the present invention there is provided a composition of matter comprising a scaffold, wherein an outer surface of the scaffold comprises a coating of nanoparticles and wherein more than 50 % of the metal nanoparticles are positioned on the outer surface of the scaffold.

As used herein, the term "scaffold" refers to a 3 dimensional matrix upon which cells may be cultured (i.e., survive and preferably proliferate for a predetermined time period).

The scaffold may be a sponge, hydrogel, acellular matrix and combinations thereof, as further described below.

The scaffold of the present invention may be made uniformly of a single polymer, co-polymer or blend thereof. However, it is also possible to form a scaffold according to the invention of a plurality of different polymers. There are no particular limitations to the number or arrangement of polymers used in forming the scaffold. Any combination which is biocompatible, may be formed into fibers, and degrades at a suitable rate, may be used.

Both the choice of polymer and the ratio of polymers in a co-polymer may be adjusted to optimize the stiffness of the scaffold. The molecular weight and cross-link density of the scaffold may also be regulated to control both the mechanical properties of the scaffold and the degradation rate (for degradable scaffolds). The mechanical properties may also be optimized to mimic those of the tissue at the implant site. The shape and size of the final scaffold should be adapted for the implant site and tissue type.

Scaffold material may comprise natural or synthetic organic polymers that can be gelled, or polymerized or solidified (e.g., by aggregation, coagulation, hydrophobic interactions, or cross-linking) into a 3-D open-lattice structure that entraps water or other molecules, e.g., to form a hydrogel. Structural scaffold materials may comprise a single polymer or a mixture of two or more polymers in a single composition. Additionally, two or more structural scaffold materials may be co-deposited so as to form a polymeric mixture at the site of deposition. Polymers used in scaffold material compositions may be biocompatible, biodegradable and/or bioerodible and may act as adhesive substrates for cells. In exemplary embodiments, structural scaffold materials are easy to process into complex shapes and have a rigidity and mechanical strength suitable to maintain the desired shape under in vivo conditions.

In certain embodiments, the structural scaffold materials may be non-resorbing or non-biodegradable polymers or materials.

The phrase "non-biodegradable polymer", as used herein, refers to a polymer or polymers which at least substantially (i.e. more than 50 %) do not degrade or erode in vivo. The terms "non-biodegradable" and "non-resorbing" are equivalent and are used interchangeably herein.

Such non-resorbing scaffold materials may be used to fabricate materials which are designed for long term or permanent implantation into a host organism. In exemplary embodiments, non-biodegradable structural scaffold materials may be biocompatible. Examples of biocompatible non-biodegradable polymers which are useful as scaffold materials include, but are not limited to, polyethylenes, polyvinyl chlorides, polyamides such as nylons, polyesters, rayons, polypropylenes, polyacrylonitriles, acrylics, polyisoprenes, polybutadienes and polybutadiene-polyisoprene copolymers, neoprenes and nitrile rubbers, polyisobutylenes, olefinic rubbers such as ethylene-propylene rubbers, ethylene-propylene-diene monomer rubbers, and polyurethane elastomers, silicone rubbers, fluoroelastomers and fluorosilicone rubbers, homopolymers and copolymers of vinyl acetates such as ethylene vinyl acetate copolymer, homopolymers and copolymers of acrylates such as polymethylmethacrylate, polyethylmethacrylate, polymethacrylate, ethylene glycol dimethacrylate, ethylene dimethacrylate and hydroxymethyl methacrylate, polyvinylpyrrolidones, polyacrylonitrile butadienes, polycarbonates, polyamides, fluoropolymers such as polytetrafluoroethylene and polyvinyl fluoride, polystyrenes, homopolymers and copolymers of styrene acrylonitrile, cellulose acetates, homopolymers and copolymers of acrylonitrile butadiene styrene, polymethylpentenes, polysulfones, polyesters, polyimides, polyisobutylenes, polymethylstyrenes, and other similar compounds known to those skilled in the art.

In other embodiments, the structural scaffold materials may be a "bioerodible" or "biodegradable" polymer or material.

The phrase "biodegradable polymer" as used herein, refers to a polymer or polymers which degrade in vivo, and wherein erosion of the polymer or polymers over time occurs concurrent with or subsequent to release of the islets. The terms "biodegradable" and "bioerodible" are equivalent and are used interchangeably herein.

Such bioerodible or biodegradable scaffold materials may be used to fabricate temporary structures. In exemplary embodiments, biodegradable or bioerodible structural scaffold materials may be biocompatible. Examples of biocompatible biodegradable polymers which are useful as scaffold materials include, but are not limited to, polylactic acid, polyglycolic acid, polycaprolactone, and copolymers thereof, polyesters such as polyglycolides, polyanhydrides, polyacrylates, polyalkyl cyanoacrylates such as n-butyl cyanoacrylate and isopropyl cyanoacrylate, polyacrylamides, polyorthoesters, polyphosphazenes, polypeptides, polyurethanes, polystyrenes, polystyrene sulfonic acid, polystyrene carboxylic acid, polyalkylene oxides, alginates, agaroses, dextrins, dextrans, polyanhydrides, biopolymers such as collagens and elastin, alginates, chitosans, glycosaminoglycans, and mixtures of such polymers. In still other embodiments, a mixture of non-biodegradable and bioerodible and/or biodegradable scaffold materials may be used to form a biomimetic structure of which part is permanent and part is temporary.

PLA, PGA and PLA/PGA copolymers are particularly useful for forming the scaffolds of the present invention. PLA polymers are usually prepared from the cyclic esters of lactic acids. Both L(+) and D(-) forms of lactic acid can be used to prepare the PLA polymers, as well as the optically inactive DL-lactic acid mixture of D(-) and L(+) lactic acids. PGA is the homopolymer of glycolic acid (hydroxyacetic acid). In the conversion of glycolic acid to poly(glycolic acid), glycolic acid is initially reacted with itself to form the cyclic ester glycolide, which in the presence of heat and a catalyst is converted to a high molecular weight linear-chain polymer. The erosion of the polyester scaffold is related to the molecular weights. The higher molecular weights, weight average molecular weights of 90,000 or higher, result in polymer scaffolds which retain their structural integrity for longer periods of time; while lower molecular weights, weight average molecular weights of 30,000 or less, result in both slower release and shorter scaffold lives. For example, poly(lactide-co-glycolide) (50:50) degrades in about six weeks following implantation.

According to a embodiment of this aspect of the present invention the scaffold comprises polycaprolactone (PCL).

According to still another embodiment of this aspect of the present invention the scaffold comprises polycaprolactone (PCL) and gelatin.

According to one embodiment of this aspect of the present invention the scaffold comprises a 50:50 mixture of poly(L-lactic acid) and poly(lactic acid-co-glycolic acid.

According to one embodiment of this aspect of the present invention the scaffold comprises poly(lactic acid-co-glycolic acid.

In certain embodiments, the structural scaffold material composition is solidified or set upon exposure to a certain temperature; by interaction with ions, e.g., copper, calcium, aluminum, magnesium, strontium, barium, tin, and di-, tri- or tetra-functional organic cations, low molecular weight dicarboxylate ions, sulfate ions, and carbonate ions; upon a change in pH; or upon exposure to radiation, e.g., ultraviolet or visible light. In an exemplary embodiment, the structural scaffold material is set or solidified upon exposure to the body temperature of a mammal, e.g., a human being. The scaffold material composition can be further stabilized by cross-linking with a polyion.

In an exemplary embodiment, scaffold materials may comprise naturally occurring substances, such as, fibrinogen, fibrin, thrombin, chitosan, collagen, alginate, poly(N-isopropylacrylamide), hyaluronate, albumin, collagen, synthetic polyamino acids, prolamines, polysaccharides such as alginate, heparin, and other naturally occurring biodegradable polymers of sugar units.

In certain embodiments, structural scaffold materials may be ionic hydrogels, for example, ionic polysaccharides, such as alginates or chitosan. Ionic hydrogels may be produced by cross-linking the anionic salt of alginic acid, a carbohydrate polymer isolated from seaweed, with ions, such as calcium cations. The strength of the hydrogel increases with either increasing concentrations of calcium ions or alginate. For example, U.S. Pat. No. 4,352,883 describes the ionic cross-linking of alginate with divalent cations, in water, at room temperature, to form a hydrogel matrix. In general, these polymers are at least partially soluble in aqueous solutions, e.g., water, or aqueous alcohol solutions that have charged side groups, or a monovalent ionic salt thereof. There are many examples of polymers with acidic side groups that can be reacted with cations, e.g., poly(phosphazenes), poly(acrylic acids), and poly(methacrylic acids). Examples of acidic groups include carboxylic acid groups, sulfonic acid groups, and halogenated (preferably fluorinated) alcohol groups. Examples of polymers with basic side groups that can react with anions are poly(vinyl amines), poly(vinyl pyridine), and poly(vinyl imidazole). Polyphosphazenes are polymers with backbones consisting of nitrogen and phosphorous atoms separated by alternating single and double bonds. Each phosphorous atom is covalently bonded to two side chains. Polyphosphazenes that can be used have a majority of side chains that are acidic and capable of forming salt bridges with di- or trivalent cations. Examples of acidic side chains are carboxylic acid groups and sulfonic acid groups. Bioerodible polyphosphazenes have at least two differing types of side chains, acidic side groups capable of forming salt bridges with multivalent cations, and side groups that hydrolyze under in vivo conditions, e.g., imidazole groups, amino acid esters, glycerol, and glucosyl. Bioerodible or biodegradable polymers, i.e., polymers that dissolve or degrade within a period that is acceptable in the desired application (usually in vivo therapy), will degrade in less than about five years or in less than about one year, once exposed to a physiological solution of pH 6-8 having a temperature of between about 25.degree. C. and 38.degree. C. Hydrolysis of the side chain results in erosion of the polymer. Examples of hydrolyzing side chains are unsubstituted and substituted imidizoles and amino acid esters in which the side chain is bonded to the phosphorous atom through an amino linkage.

Typically, the scaffolds of the present invention are porous. The porosity of the scaffold may be controlled by a variety of techniques known to those skilled in the art. The minimum pore size and degree of porosity is dictated by the need to provide enough room for the cells and for nutrients to filter through the scaffold to the cells. The maximum pore size and porosity is limited by the ability of the scaffold to maintain its mechanical stability after seeding. As the porosity is increased, use of polymers having a higher modulus, addition of stiffer polymers as a co-polymer or mixture, or an increase in the cross-link density of the polymer may all be used to increase the stability of the scaffold with respect to cellular contraction.

The scaffolds may be made by any of a variety of techniques known to those skilled in the art. Salt-leaching, porogens, solid-liquid phase separation (sometimes termed freeze-drying), and phase inversion fabrication may all be used to produce porous scaffolds. Fiber pulling and weaving (see, e.g. Vacanti, et al., (1988) Journal of Pediatric Surgery, 23: 3-9) may be used to produce scaffolds having more aligned polymer threads. Those skilled in the art will recognize that standard polymer processing techniques may be exploited to create polymer scaffolds having a variety of porosities and microstructures.

According to a particular embodiment, the scaffold is made by electro spinning.

As used herein, the term "electrospinning" refers to a technology which produces electrospun fibers (e.g. nanofibers) from a polymer solution. During this process, one or more polymers are liquefied (i.e. melted or dissolved) and placed in a dispenser. An electrostatic field is employed to generate a positively charged jet from the dispenser to the collector. Thus, a dispenser (e.g., a syringe with metallic needle) is typically connected to a source of high voltage, preferably of positive polarity, while the collector is grounded, thus forming an electrostatic field between the dispenser and the collector. Alternatively, the dispenser can be grounded while the collector is connected to a source of high voltage, preferably with negative polarity. As will be appreciated by one ordinarily skilled in the art, any of the above configurations establishes motion of positively charged jet from the dispenser to the collector. Reverse polarity for establishing motions of a negatively charged jet from the dispenser to the collector is also contemplated. At the critical voltage, the charge repulsion begins to overcome the surface tension of the liquid drop. The charged jets depart from the dispenser and travel within the electrostatic field towards the collector. Moving with high velocity in the inter-electrode space, the jet stretches and the solvent therein evaporates, thus forming fibers which are collected on the collector forming the electro spun scaffold.

Several parameters may affect the diameter of the fiber, these include, the size of the dispensing hole of the dispenser, the dispensing rate, the strength of the electrostatic field, the distance between the dispenser and/or the concentration of the polymer used for fabricating the electro spun fiber.

According to a particular embodiment, the average fiber diameter is between 20 nm - 10µm, 20 - 1µm, 100-300 nm, e.g. about 250 nm.

The dispenser can be, for example, a syringe with a metal needle or a bath provided with one or more capillary apertures from which the liquefied polymer(s) can be extruded, e.g., under the action of hydrostatic pressure, mechanical pressure, air pressure and high voltage.

According to one embodiment, the collector is a rotating collector which serves for collecting the electrospun scaffold thereupon. Employing a rotating collector can result in an electrospun scaffold with a continuous gradient of porosity. Such a porosity gradient can be achieved by continuous variation in the velocity of the collector or by a longitudinal motion of the dispenser, as disclosed for example in WO06106506, these result in a substantial variation in the density and/or spatial distribution of the fibers on the collector and thus, result in a porosity gradient along the radial direction or along the longitudinal direction of the collector, respectively. Typically, but not obligatorily, the rotating collector has a cylindrical shape (e.g., a drum), however, it will be appreciated that the rotating collector can be also of a planar geometry.

According to another embodiment, the collector is a flat ground collector which serves for collecting the electrospun scaffold thereupon. Employing a flat ground collector enables collection of random nanofibers. It will be appreciated that the flat ground collector is typically a horizontal collector or a vertical collector.

The polymer used to fabricate the scaffolds of the present invention can be natural, synthetic, biocompatible, biodegradable and/or non-biodegradable polymers, as further described herein above.

According to another exemplary embodiment, adhesive agents are included in the scaffolds (e.g. electrospun scaffolds) of the present invention. Such adhesive agents may be used to unite or bond the electrospun polymers together.

Such an adhesive agent may include, without being limited to, gelatin, fibrin, fibronectin, collagen or RGB. Ratios of adhesive agents (e.g. gelatin): polymer may be about 50:50, may be about 40:60, may be about 30:70, may be about 20:80, or may be about 10:90.

According to a particular embodiment, the scaffold comprises spring-like fibers (i.e. coiled fibers), that are also able to stretch with extensibility over 200%, and recoil back to the same position. Such fibers can be generated by electrospinning by fixing the rate of delivery of the polymer solution through the capillary. Thus, for example, the present inventors have shown that spring-like fibers can be generated if a polymer solution of PCL (e.g. at concentrations between 10% to 17.5% w/v%) dissolved in dichloromethane(DCM) and dimethylformamide (DMF) (for examples in ratios of 3:1, 2:1, 1:1 and 1:2) through a capillary at a rate of 0.1-20 ml/h (e.g. 0.5ml/h).

Spring-like fibers can also be generated using PLGA.

Scaffold materials are readily available to one of ordinary skill in the art, usually in the form of a solution (suppliers are, for example, BDH, United Kingdom, and Pronova Biomedical Technology a.s. Norway). For a general overview of the selection and preparation of scaffolding materials, see the American National Standards Institute publication No. F2064-00 entitled Standard Guide for Characterization and Testing of Alginates as Starting Materials Intended for Use in Biomedical and Tissue Engineering Medical Products Applications".

According to still another embodiment, the scaffold is generated from decellularized tissue and more specifically from decellularized extracellular matrix (ECM) of a tissue.

As used herein the phrase "decellularized ECM of a tissue" refers to the extracellular matrix which supports tissue organization (e.g., a natural tissue) and underwent a decellularization process (*i.e.,* a removal of all cells from the tissue) and is thus completely devoid of any cellular components.

The phrase "completely devoid of any cellular components" as used herein refers to being more than 90 %, 91 %, 92 %, 93 %, 94 %, 95 %, 96 %, 97 %, 98 %, 99 %, (e.g., 100 %) devoid of the cellular components present in the natural (e.g., native) tissue. As used herein, the phrase "cellular components" refers to cell membrane components or intracellular components which make up the cell. Examples of cell components include cell structures (e.g., organelles) or molecules comprised in same. Examples of such include, but are not limited to, cell nuclei, nucleic acids, residual nucleic acids (e.g., fragmented nucleic acid sequences), cell membranes and/or residual cell membranes (e.g., fragmented membranes) which are present in cells of the tissue. It will be appreciated that due to the removal of all cellular components from the tissue, such a decellularized matrix cannot induce an immunological response when implanted in a subject.

The phrase "extracellular matrix (ECM)" as used herein, refers to a complex network of materials produced and secreted by the cells of the tissue into the surrounding extracellular space and/or medium and which typically together with the cells of the tissue impart the tissue its mechanical and structural properties. Generally, the ECM includes fibrous elements (particularly collagen, elastin, or reticulin), cell adhesion polypeptides (e.g., fibronectin, laminin and adhesive glycoproteins), and space-filling molecules [usually glycosaminoglycans (GAG), proteoglycans].

A tissue-of-interest (e.g., pancreas, myocardium, omentum) may be derived from an autologous or non-autologous tissue (e.g., allogeneic or even xenogeneic tissue, due to non-immunogenicity of the resultant decellularized matrix). The tissue is removed from the subject [e.g., an animal, preferably a mammal, such as a pig, monkey or chimpanzee, or alternatively, a deceased human being (shortly after death)] and can be washed e.g. in a sterile saline solution (0.9 % NaCl, pH = 7.4) or phosphate buffered saline (PBS), which can be supplemented with antibiotics such as Penicillin/Streptomycin 250 units/ml. Although whole tissues can be used, for several applications segments of tissues may be cut e.g. sliced. Such tissue segments can be of various dimensions, depending on the original tissue used and the desired application.

Methods of decellularizing tissue are known in the art such as those disclosed in U.S. Patent Application No. 20120156250, U.S. Patent Application No. 201000267143, PCT Application No. WO2014/037942, U.S. Patent Application No. 61/838,428, International Patent Application No. WO2009/085547 and U.S. Patent Application No. 20050013870.

The outer surface of the scaffolds described herein comprise a coating of metal nanoparticles, wherein more than 50 % of the metal nanoparticles are positioned on the outer surface of the scaffold. According to another embodiment, more than 60 % of the metal nanoparticles are positioned on the outer surface of the scaffold. According to another embodiment, more than 70 % of the metal nanoparticles are positioned on the outer surface of the scaffold. According to yet another embodiment, more than 80 % of the metal nanoparticles are positioned on the outer surface of the scaffold. According to still another embodiment of the present invention, more than 90 % of the metal nanoparticles are positioned on the outer surface of the scaffold.

It will be appreciated that in order for the metal nanoparticles to be situated on the outer surface of the scaffold, typically the scaffold is coated with the metal nanoparticles after it has been fabricated, and not during the fabrication process.

Examples of metal nanoparticles include, but are not limited to Al, Au, Ti, Ni, Ag, Cr, 80%Ni20%, Cr, Pd, Al, Mo, Nb, Cu, Pt, Ag, Nb and Co.

The gold nanoparticles may consist entirely of gold or may comprise additional components such as those described in U.S. Patent Application 20100106233.

According to an embodiment of this aspect of the present invention, the nanoparticles (e.g. gold nanoparticles) are not crosslinked on to the surface of the scaffolds.

The scaffolds are coated with metal nanoparticles by evaporation.

Following evaporation, the metal nanoparticles bind to the surface of the scaffolds by physical conjugation, as high energy metal vapor cools down on the fibers. Later, evaporated metal bonds with ligands such as oxygen and sulfur on the scaffold surface.

The coating is between 2-20 nm in thickness and more preferably between 4-14 nm in thickness.

Typically, the coating is spread non-homogeneously over the scaffold - i.e. is discontinuous, forming islands of coated scaffold and islands of non-coated scaffolds. Preferably, at least 10 % of the scaffold is coated with nanoparticles, at least 20 % of the scaffold is coated with nanoparticles at least 30 % of the scaffold is coated with nanoparticles, at least 40 % of the scaffold is coated with nanoparticles, at least 50 % of the scaffold is coated with nanoparticles, at least 60 % of the scaffold is coated with nanoparticles, at least 70 % of the scaffold is coated with nanoparticles.

Preferably, no more than 95 % of the scaffold is coated with the nanoparticles, no more than 94 % of the scaffold is coated with the nanoparticles, no more than 93 % of the scaffold is coated with the nanoparticles, no more than 92 % of the scaffold is coated with the nanoparticles, no more than 91 % of the scaffold is coated with the nanoparticles, no more than 90 % of the scaffold is coated with the nanoparticles.

Therapeutic compounds or agents that modify cellular activity can also be incorporated (e.g. attached to, coated on, embedded or impregnated) into the scaffold material. Such compounds and agents may be incorporated directly onto the scaffold and/or onto the metal (e.g. gold) nanoparticles themselves. Campbell et al (US Patent Application No. 20030125410) discloses methods for fabrication of 3D scaffolds for stem cell growth, the scaffolds having preformed gradients of therapeutic compounds. The scaffold materials, according to Campbell et al, fall within the category of "bio-inks". Such "bio-inks" are suitable for use with the compositions and methods of the present invention.

Exemplary agents that may be incorporated into or on the scaffold of the present invention include, but are not limited to those that promote cell adhesion (e.g. fibronectin, integrins), cell colonization, cell proliferation, cell differentiation, cell extravasation and/or cell migration. Thus, for example, the agent may be an amino acid, a small molecule chemical, a peptide, a polypeptide, a protein, a DNA, an RNA, a lipid and/or a proteoglycan.

According to another embodiment an antibody is incorporated on to the scaffold of the present invention. Preferably the antibody recognizes a cell-surface marker or a cell-secreted agent.

Proteins that may be incorporated into, or on the surface of the scaffolds of the present invention include, but are not limited to extracellular matrix proteins, cell adhesion proteins, growth factors, cytokines, hormones, proteases and protease substrates. Thus, exemplary proteins include vascular endothelial-derived growth factor (VEGF), activin-A, retinoic acid, epidermal growth factor, bone morphogenetic protein, TGFβ, hepatocyte growth factor, platelet-derived growth factor (PDGF), TGFα, IGF-I and II, hematopoetic growth factors, heparin binding growth factor, peptide growth factors, erythropoietin, interleukins, tumor necrosis factors, interferons, colony stimulating factors, basic and acidic fibroblast growth factors, nerve growth factor (NGF) or muscle morphogenic factor (MMP). The particular growth factor employed should be appropriate to the desired cell activity. The regulatory effects of a large family of growth factors are well known to those skilled in the art.

An exemplary scaffold contemplated by the present inventors is a scaffold comprising fibers of decellularized extracellular matrix (ECM), wherein an outer surface of the scaffold comprises a coating of metal nanoparticles, with the proviso that the metal nanoparticles (e.g. gold nanoparticles) are not crosslinked to the scaffold.

Another exemplary scaffold contemplated by the present inventors is a scaffold comprising decellularized omentum, wherein an outer surface of the scaffold comprises a coating of metal nanoparticles (e.g. gold nanoparticles).

The scaffolds of the present invention may also comprise cells (i.e. biological cells).

The cells comprise electrically excitable cells such as cardiac cell (e.g. cardiomyocyte), glandular cells and nerve cells.

As used herein, the term "cardiomyocytes" refers to fully or at least partially differentiated cardiomyocytes. Thus, cardiomyocytes may be derived from stem cells (such as embryonic stem cells or adult stem cells, such as mesenchymal stem cells). Methods of differentiating stem cells along a cardiac lineage are well known in the art - [Muller-Ehmsen J, et al., Circulation. 2002;105:1720-6; Zhang M, et al., J Mol Cell Cardiol. 2001;33:907-21, Xu et al, Circ Res. 2002;91:501-508, and U.S. Pat. Appl. No. 20050037489].

According to one embodiment the cardiomyocytes of the present invention are at least capable of spontaneous contraction. According to another embodiment, the cardiomyocytes of the present invention express at least one marker (more preferably at least two markers and even more preferably at least three markers) of early-immature cardiomyocytes (e.g. atrial natriuretic factor (ANF), Nkx2.5, MEF2C and α-skeletal actin). According to another embodiment, the cardiomyocytes of the present invention express at least one marker (more preferably at least two markers and even more preferably at least three markers) of fully differentiated cardiomyocytes (e.g. MLC-2V, α-MHC, α-cardiac actin and Troponin I).

Screening of partially differentiated cardiomyocytes may be performed by a method enabling detection of at least one characteristic associated with a cardiac phenotype, as described hereinbelow, for example via detection of cardiac specific mechanical contraction, detection of cardiac specific structures, detection of cardiac specific proteins, detection of cardiac specific RNAs, detection of cardiac specific electrical activity, and detection of cardiac specific changes in the intracellular concentration of a physiological ion.

Additional cells that may be seeded together with cardiomyoctyes include for example endothelial cells and/or fibroblast cells (which may or may not be derived from cardiac tissue). Accordingly, a pool of cardiomyocytes, endothelial cells and fibroblasts (in the presence or absence of an appropriate gel, as described herein below) may be generated and seeded onto the scaffold.

Seeding of the cells on the scaffolds is a critical step in the establishment of an engineered tissue. Since it has been observed that the initial distribution of cells within the scaffold after seeding is related to the cell densities subsequently achieved, methods of cell seeding require careful consideration. Thus, cells can be seeded in a scaffold by static loading, or by seeding in stirred flask bioreactors (scaffold is typically suspended from a solid support), in a rotating wall vessel, or using direct perfusion of the cells in medium in a bioreactor. Highest cell density throughout the scaffold is achieved by the latter (direct perfusion) technique.

The cells may be seeded directly onto the scaffold, or alternatively, the cells may be mixed with a gel which is then absorbed onto the interior and exterior surfaces of the scaffold and which may fill some of the pores of the scaffold. Capillary forces will retain the gel on the scaffold before hardening, or the gel may be allowed to harden on the scaffold to become more self-supporting. Alternatively, the cells may be combined with a cell support substrate in the form of a gel optionally including extracellular matrix components. An exemplary gel is Matrigel™, from Becton-Dickinson. Matrigel™ is a solubilized basement membrane matrix extracted from the EHS mouse tumor (Kleinman, H. K., et al., Biochem. 25:312, 1986). The primary components of the matrix are laminin, collagen I, entactin, and heparan sulfate proteoglycan (perlecan) (Vukicevic, S., et al., Exp. Cell Res. 202:1, 1992). Matrigel™ also contains growth factors, matrix metalloproteinases (MMPs [collagenases]), and other proteinases (plasminogen activators [PAs]) (Mackay, A. R., et al., BioTechniques 15:1048, 1993). The matrix also includes several undefined compounds (Kleinman, H. K., et al., Biochem. 25:312, 1986; McGuire, P. G. and Seeds, N. W., J. Cell. Biochem. 40:215, 1989), but it does not contain any detectable levels of tissue inhibitors of metalloproteinases (TIMPs) (Mackay, A. R., et al., BioTechniques 15:1048, 1993). Alternatively, the gel may be growth-factor reduced Matrigel, produced by removing most of the growth factors from the gel (see Taub, et al., Proc. Natl. Acad. Sci. USA (1990); 87 (10:4002-6). In another embodiment, the gel may be a collagen I gel, alginate, or agar. Such a gel may also include other extracellular matrix components, such as glycosaminoglycans, fibrin, fibronectin, proteoglycans, and glycoproteins. The gel may also include basement membrane components such as collagen IV and laminin. Enzymes such as proteinases and collagenases may be added to the gel, as may cell response modifiers such as growth factors and chemotactic agents.

Following seeding, the scaffold-supported cells may be cultured under conditions which allow the formation of 3D structures. According to one embodiment, the scaffold is cultured under conditions which allow formation of endothelial structures within the scaffold.

The scaffold-supported cells may be cultured for 1 day, two days, three days, four days, five days, six days, at least 1 week, 10 days or two weeks, three weeks, four weeks.

The scaffolds described herein (either alone or seeded with cells) may be used for modifying the electrophysiological function of excitable tissues. As is further detailed in the Examples section which follows, the present invention can be utilized to restore enhance or suppress electrophysiological function across a tissue region thereby treating diseases caused by dysfunction in, or damage to, excitable tissues.

As used herein the term "treating" refers to inhibiting or arresting the development of a disease, disorder or condition and/or causing the reduction, remission, or regression of a disease, disorder or condition in a subject suffering from, being predisposed to, or diagnosed with, the disease, disorder or condition. Those of skill in the art will be aware of various methodologies and assays which can be used to assess the development of a disease, disorder or condition, and similarly, various methodologies and assays which can be used to assess the reduction, remission or regression of a disease, disorder or condition.

Examples of diseases and conditions which can be treated using the scaffolds of the present invention include, but are not limited to glucose regulation disorders (e.g., diabetes mellitus), cardiac arrythmia (e.g., bradycardia, atrial fibrillation) diseases which are treatable by modulating (i.e., increasing or decreasing) the refractory period of a cardiac tissue (e.g., atrial fibrillation, atrial flutter, atrial tachycardia and ventricular tachycardia), diseases which are treatable by modulating (i.e., increasing or decreasing) neural excitability (e.g., epilepsy, Parkinson's disease and Alzheimer's disease) and diseases and conditions which are treatable by modulating (i.e., increasing or decreasing) pyramidal or purkinje cell coupling (e.g., cerebrovascular accident, epilepsy and pain (e.g., phantom pain).

According to a particular embodiment, the disorder is a cardiac disorder which is associated with a defective or absent myocardium.

Thus, there is provided a method of treating cardiac disorder associated with a defective or absent myocardium in a subject, the method comprising transplanting a therapeutically effective amount of the compositions of the present invention into the subject.

The method may be applied to repair cardiac tissue in a human subject having a cardiac disorder so as to thereby treat the disorder. The method can also be applied to repair cardiac tissue susceptible to be associated with future onset or development of a cardiac disorder so as to thereby inhibit such onset or development.

The present invention can be advantageously used to treat disorders associated with, for example, necrotic, apoptotic, damaged, dysfunctional or morphologically abnormal myocardium. Such disorders include, but are not limited to, ischemic heart disease, cardiac infarction, rheumatic heart disease, endocarditis, autoimmune cardiac disease, valvular heart disease, congenital heart disorders, cardiac rhythm disorders, impaired myocardial conductivity and cardiac insufficiency. Since the majority of cardiac diseases involve necrotic, apoptotic, damaged, dysfunctional or morphologically abnormal myocardium, and since the vascularized cardiac tissue of the present invention displays a highly differentiated, highly functional, and proliferating cardiomyocytic phenotype, the method of repairing cardiac tissue of the present invention can be used to treat the majority of instances of cardiac disorders.

The method according to this aspect of the present invention can be advantageously used to efficiently reverse, inhibit or prevent cardiac damage caused by ischemia resulting from myocardial infarction.

The method according to this aspect of the present invention can be used to treat cardiac disorders characterized by abnormal cardiac rhythm, such as, for example, cardiac arrhythmia.

As used herein the phrase "cardiac arrhythmia" refers to any variation from the normal rhythm of the heart beat, including, but not limited to, sinus arrhythmia, premature heat, heart block, atrial fibrillation, atrial flutter, pulsus alternans and paroxysmal tachycardia.

The method according to this aspect of the present invention can be used to treat impaired cardiac function resulting from tissue loss or dysfunction that occur at critical sites in the electrical conduction system of the heart, that may lead to inefficient rhythm initiation or impulse conduction resulting in abnormalities in heart rate.

The method according to this aspect of the present invention is effected by transplanting a therapeutically effective dose of the composition of the present invention to the heart of the subject, preferably by injection into the heart of the subject.

As used herein, "transplanting" refers to providing the scaffold of the present invention (with or without cells), using any suitable route.

As used herein, a therapeutically effective dose is an amount sufficient to effect a beneficial or desired clinical result, which dose could be administered in one or more administration. According to one embodiment, a single administration is employed. The injection can be administered into various regions of the heart, depending on the type of cardiac tissue repair required. Intramyocardial administration is particularly advantageous for repairing cardiac tissue in a subject having a cardiac disorder characterized by cardiac arrhythmia, impaired, cardiac conducting tissue or myocardial ischemia.

Such transplantation directly into cardiac tissue ensures that the administered cells/tissues will not be lost due to the contracting movements of the heart.

The compositions of the present invention can be transplanted via transendocardial or transepicardial injection, depending on the type of cardiac tissue repair being effected, and the physiological context in which the cardiac repair is effected. This allows the administered cells or tissues to penetrate the protective layers surrounding membrane of the myocardium.

Preferably, a catheter-based approach is used to deliver a transendocardial injection. The use of a catheter precludes more invasive methods of delivery wherein the opening of the chest cavity would be necessitated.

In the case of repairing cardiac tissue in a subject having a cardiac disorder characterized by cardiac arrhythmia, electrophysiological mapping of the heart and/or inactivation of cardiac tissue by radiofrequency treatment may be advantageously performed in combination with administration of the scaffolds of the present invention if needed.

To repair cardiac tissue damaged by ischemia, for example due to a cardiac infarct, the scaffold of the present invention is preferably administered to the border area of the infarct. As one skilled in the art would be aware, the infarcted area is grossly visible, allowing such specific localization of application of therapeutic cells to be possible. The precise determination of an effective dose in this particular case may depend, for example, on the size of an infarct, and the time elapsed following onset of myocardial ischemia.

Transplantation of the scaffold of the present invention for repair of damaged myocardium is effected following sufficient reduction of inflammation of affected cardiac tissues and prior to formation of excessive scar tissue.

The present invention can be used to generate cardiomyocytic cells and tissues displaying a desired proliferative capacity, thus cells and tissues are preferably selected displaying a suitable proliferative capacity for administration, depending on the type of cardiac tissue repair being effected. Administration of highly proliferative cells may be particularly advantageous for reversing myocardial damage resulting from ischemia since, as previously described, it is the essential inability of normal adult cardiomyocytes to proliferate which causes the irreversibility of ischemia induced myocardial damage.

Since porcine models are widely considered to be excellent models for human therapeutic protocols and since such models have been widely employed and characterized, it is well within the grasp of the ordinarily skilled artisan to determine a therapeutically effective dose for a human based on the guidance provided herein, and on that provided by the extensive literature of the art.

Determination of an effective dose is typically effected based on factors individual to each subject, including, for example, weight, age, physiological status, medical history, and parameters related to the cardiac disorder, such as, for example, infarct size and elapsed time following onset of ischemia. One skilled in the art, specifically a cardiologist, would be able to determine the amount and number of cells comprised in the composition of the present invention that would constitute an effective dose, and the optimal mode of administration thereof without undue experimentation.

It will be recognized by the skilled practitioner that when administering non-syngeneic cells or tissues to a subject, there is routinely immune rejection of such cells or tissues by the subject. Thus, the method of the present invention may also comprise treating the subject with an immunosuppressive regimen, preferably prior to such administration, so as to inhibit such rejection. Immunosuppressive protocols for inhibiting allogeneic graft rejection, for example via administration of cyclosporin A, immunosuppressive antibodies, and the like are widespread and standard practice in the clinic.

The scaffold of the present invention may be transplanted to a human subject per se, or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the scaffold supported cells of the present invention accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

As used herein the term "about" refers to ± 10 %.

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Various embodiments and aspects of the present invention as delineated hereinabove and as claimed in the claims section below find experimental support in the following examples.

As used herein the term "about" refers to ± 10 %.

The terms "comprises", "comprising", "includes", "including", "having" and their conjugates mean "including but not limited to".

The term "consisting of' means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### EXAMPLE 1

### Gelatin/PCL scaffolds coated with nanoparticles

### Materials and Methods

***Electrospinning:*** Electrospun matrices were fabricated as previously described³¹. Briefly, 10% Gelatin (Sigma, St. Louis, MO) and 10% PCL (Sigma, St. Louis, MO) were separately dissolved in 2,2,2 trifluoroethanol (Acros, Belgium) over night at room temperature. The next day, the solutions were mixed at a ratio of 1:1. Using a syringe pump (Harvard apparatus, Holliston, MA), the polymer solution was delivered through a stainless steel 20G capillary at a feeding rate of 0.5ml/hr. A high voltage power supply (Glassman high voltage inc.) was used to apply a 10kV potential between the capillary tip and the collector, positioned 10 cm beneath the tip.

***Gold NP preparation:*** Aluminum foils covered with electrospun fibers were mounted in a VST e-beam evaporator. Homogeneous deposition was obtained by moderate rotation of the substrate plate. Au films (2.0 to 14nm thick) were prepared by Au 99.999% evaporation from a tungsten boat at 1-3 × 10⁻⁶ torr at a deposition rate of 0.5 A·sec⁻¹.

***Reflection measurements:*** Due to the thickness of the scaffolds and therefore limited transparency, localized surface plasmon resonance (LSPR) was detected by reflectance and not absorbance. Reflection measurements were carried out using an Ocean Optics reflection probe model R00-7 VIS/NIR and quartz tungsten halogen (QTH) lamp. The data were collected using an Ocean Optics Red Tide 650 diode-array spectrophotometer (spectral range: 380-900 nm) at an angel of incidence of 90. The reflection probe was centrally placed ca. 5 mm from the top edge of the slide, at a vertical distance of 8 mm from the planar substrate. The measured spot was ca. 4 mm in diameter.

***Scanning electron microscopy:*** Quanta 200 FEG Environmental Scanning Electron Microscope (ESEM) with a field-emission gun (FEG) electron source was used. Imaging was in low vacuum and high tension of 20kV with working distance of 7.3mm.

***High resolution Transmitting Electron Microscopy:*** Cross sections were prepared by embedding the polymer in epoxy glue followed by polishing to obtain the desired size. All samples were deposited on carbon coated copper grids (SPI) for transmission electron microscopy (TEM) analysis. Images were recorded using a Philips FEI Tecnai F20 FEG-TEM.

***Cardiac cell isolation and seeding:*** Neonatal ventricle myocytes (1- to 3-day-old Sprague-Dawley rats) were harvested, and cells were isolated as described previously¹². Briefly, isolated ventricles were cut into ∼1 mm³ pieces and incubated repeatedly (6-7 times) in buffer containing collagenase type II (95 U/mL; Worthington, Lakewood, NJ) and pancreatin (0.6 mg/mL; Sigma, St. Louis, MO). After each digestion round, the mixture was centrifuged (600g, 5 min, 25 °C) and the cell pellet was re-suspended in cold M-199 medium (Biological Industries, Beit Haemek, Israel) supplemented with 0.5% (v/v) fetal calf serum (FCS, Biological Industries). The pooled cells were centrifuged and resuspended twice in culture medium (M-199 medium supplemented with 5% FCS, 0.6 mM copper sulphate pentahydrate, 0.5 mM zinc sulphate heptahydrate,500 U/mL penicillin, and 100 mg/mL streptomycin [Biological Industries]). After two rounds of preplating for 40 minutes, the cells were counted and seeded on the scaffolds, using a single droplet.

***Immunostaining:*** The cellular constructs were fixed and permeabilized in cold methanol, blocked for 8 minutes at room temperature in Super Block (ScyTek laboratories, US). After three PBST washes, the samples were incubated with primary mouse monoclonal anti α-actinin (1:750, Sigma). After incubation, the samples were washed and incubated for 1 hour with goat anti-mouse Alexa Fluor 647 (1:500, Jackson, West Grove, PA). For nuclei detection, the cells were incubated for 5 min with 5 µg/mL Hoechst 33258 (Sigma).

***Analyses of tissue function:*** Contraction of the cardiac cell constructs was recorded using an inverted microscope (Nikon Eclipse TI) and NIS element software. Contraction amplitude was analyzed using ImageJ software (NIH). Contraction rate was manually counted.

***Statistical analysis:*** Statistical analysis data are presented as means ± SEM. Univariate differences between the pristine scaffold and gold NP scaffolds were assessed with Student's t test. All analyses were performed using GraphPad Prism version 5.00 for Windows (GraphPad Software). P < 0.05 was considered significant.

### RESULTS AND DISCUSSION

Electrical coupling between cardiomyocytes is essential for engineering functional cardiac tissues³². Therefore, the goal of this example was to engineer a cellular microenvironment that encourages coupling of cardiac cells, and the formation of a contracting tissue.

In the myocardium, cardiac cells, including cardiomyocytes and fibroblasts are arranged in-between an intricate network of protein fibers, such as fibrilar collagens, ranging from 10 to several hundreds of nanometers⁵. The mesh is covered with nanoscale proteins that provide specific binding sites for cell adhesion, and cues for proper cell assembly. Here, the present inventors first focused on recapitulating the morphology of the native matrix. Fibrous scaffolds with average fiber diameter of 250 nm were fabricated by electrospinning of PCL and gelatin. PCL provided adequate mechanical properties to the fibers ²⁷, while the gelatin was used to provide adhesion sites to the cultured cells^{33,34}.

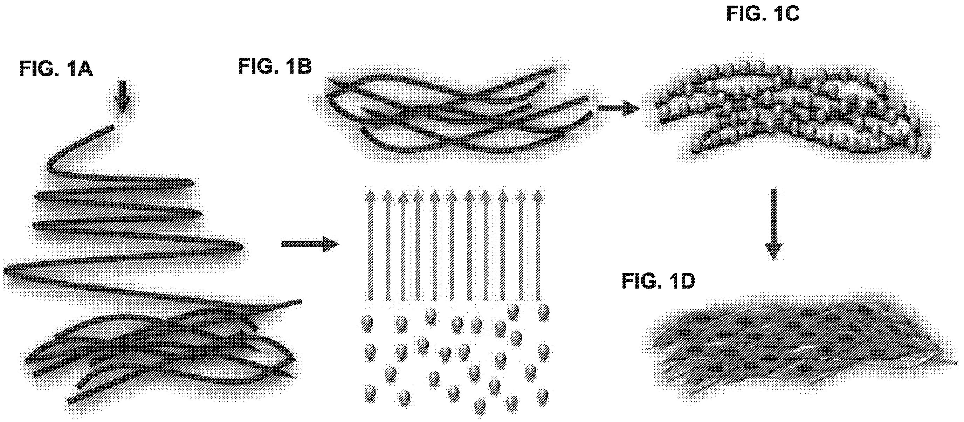

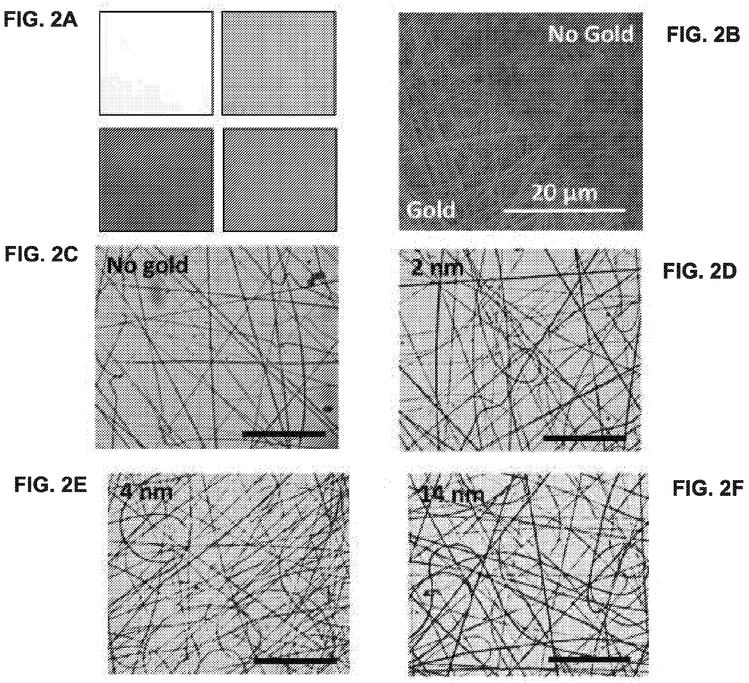

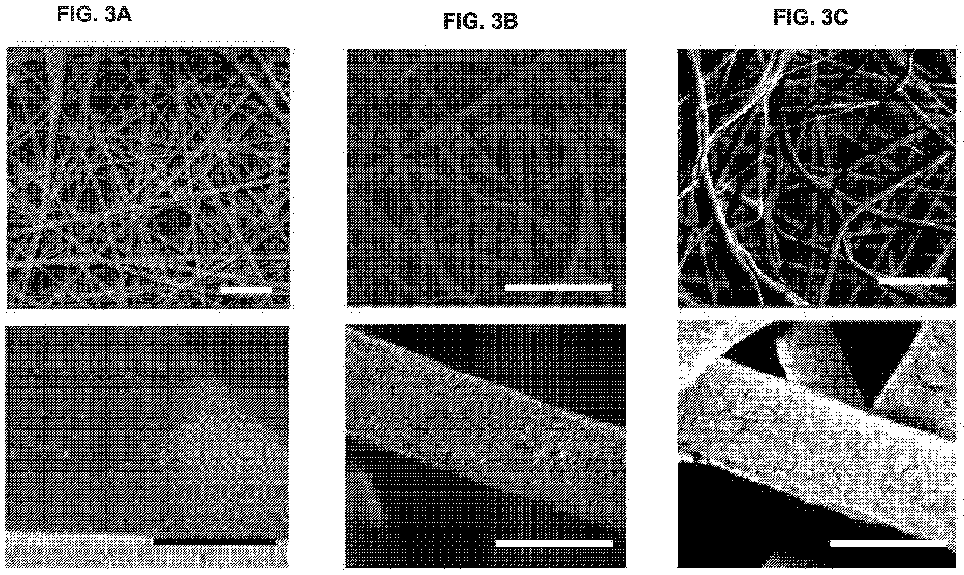

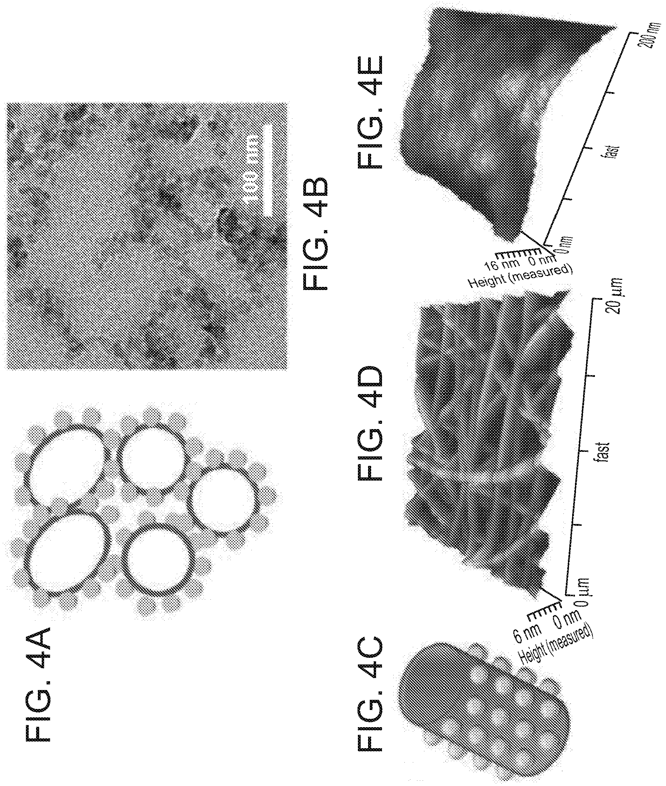

The present inventors conceived of incorporating gold NPs onto the surface of the fibers. By using this approach, they hypothesized that the NPs could serve as couplers between cells and promote functional assembly of a cardiac tissue. As a facile approach for gold incorporation to the fibers, the functional groups in the gelatin were utilized as binding sites for the evaporated gold NPs (Figure 1). By changing evaporation conditions, the present inventors were able to fabricate fibers with various nominal gold thicknesses, creating scaffolds with a typical pink to maroon color (Figure 2A). As judged by SEM and optical microscopy images, the process did not change the fibrous morphology of the scaffolds (Figure 2B-F). As shown in Figure 3, the particles were deposited homogenously on the fibers creating polymer/gold nanocomposites. Furthermore, the samples were characterized by TEM and AFM to evaluate the coverage by the particles and the composite topography (Figure 4). As shown, the particles were distributed on the fibers without affecting their fibrous morphology (Figure 4D). The scaffolds were washed several times and treated with adhesive tape to ensure strong gold NP binding.

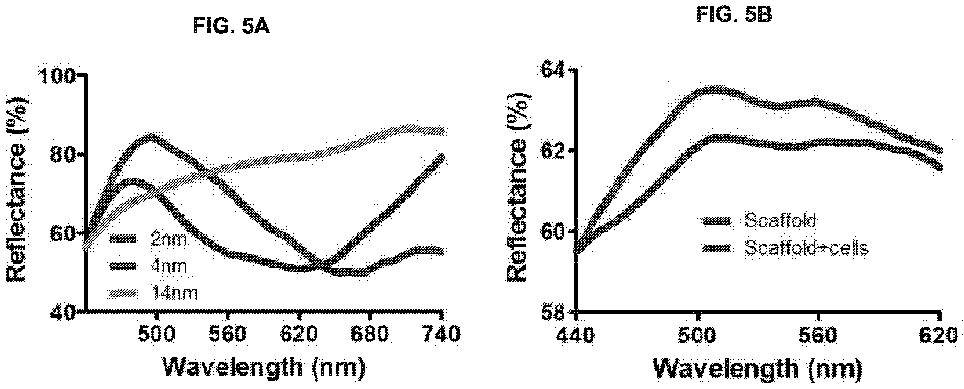

Due to the formation of small gold NPs, LSPR could be observed (Figure 5A). Thus, it is conceivable that the NPs could be utilized not only for improving the contraction of the engineered tissue, but also to monitor changes in their surrounding environment. Once the correlation between the reflectance and NP size was found, a simple spectrometer may be used to identify the size of the NPs. Since the NPs are located on the surface of the fibers (Figure 3) the LSPR could be used to observe changes in the surrounding medium, and in theory also for detecting cell adhesion or secretion of biomolecules³⁵. After seeding cells on the gold scaffolds (4 nm) a shift in the maximum reflectance of the LSPR could be observed (Figure 5B).

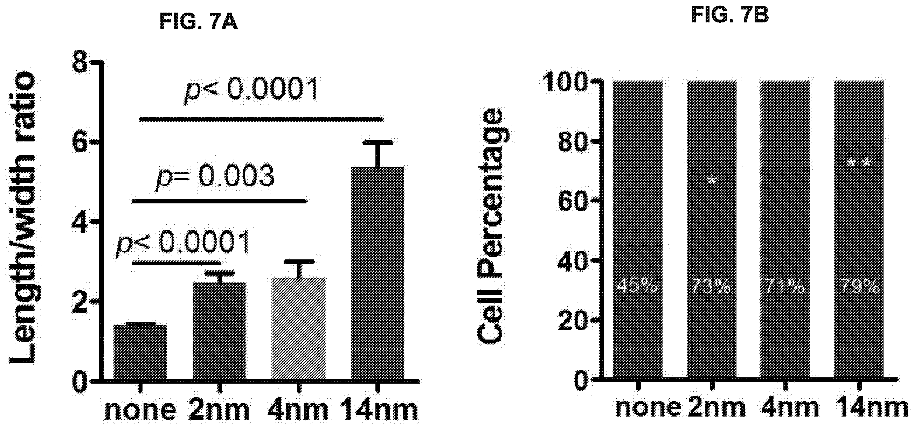

To investigate the effect of gold NPs in the scaffolds on cell and tissue assembly, cardiac cell constructs were engineered. Cardiac cells isolated from neonatal rat hearts were seeded by a single droplet on the different scaffolds. To evaluate cardiac tissue assembly, cell constructs were immunostained for cardiac sarcomeric actinin, a protein responsible for cardiac cell contraction. On day 7, cardiac cells cultured on pristine PCL/gelatin scaffolds without gold NPs, mostly exhibited a rounded morphology (Figure 6A), while cells cultured on gold NP scaffolds were elongated with massive striation, suggesting a strong contraction potential (Figure 6B-D). Furthermore, cells cultivated on the 14 nm gold NP scaffolds were aligned to each other, exhibiting morphology resembling to that of native heart bundles³⁶ (Figure 6D). Analysis of cell elongation revealed significantly higher aspect ratio (p< 0.0001) in cells grown on the gold NP fibers (Figure 7A). This phenomenon indicates that the gold NPs located on the surface of the fibers encouraged the assembly of an anisotropic tissue with typical cardiac cell morphology.

Matrix stiffness and nanostructure morphology are two parameters that have been shown to affect cellular behavior³⁷. Since cardiomyocytes, the contracting cells of the heart are terminally differentiated cells, it is essential in cardiac tissue engineering to maintain their ratio compared to non-contracting cells in the culture (i.e. fibroblasts). Otherwise, the fibroblasts can take over the culture, shifting it towards a non-contracting culture. In the present approach, the ability to control the nominal thickness of the NP and their homogeneous distribution on the fibers, may allow a facile approach for tuning the mechanical properties of the matrix according to particular needs. For example, matrix stiffness can be optimized to obtain a scaffold that on one hand may attenuate fibroblast proliferation in co-culture, and on the other, may enhance cardiomyocyte contractility performances. Thus, the ratio of cardiomyocytes to cardiac fibroblasts was measured on the different scaffolds, and evaluated the potential of the NPs to alter proliferation. Figure 7B revealed significantly higher ratio of myocytes to fibroblasts in cultures grown on gold NP fibers, suggesting a higher potential to maintain the contractility of the engineered tissue.

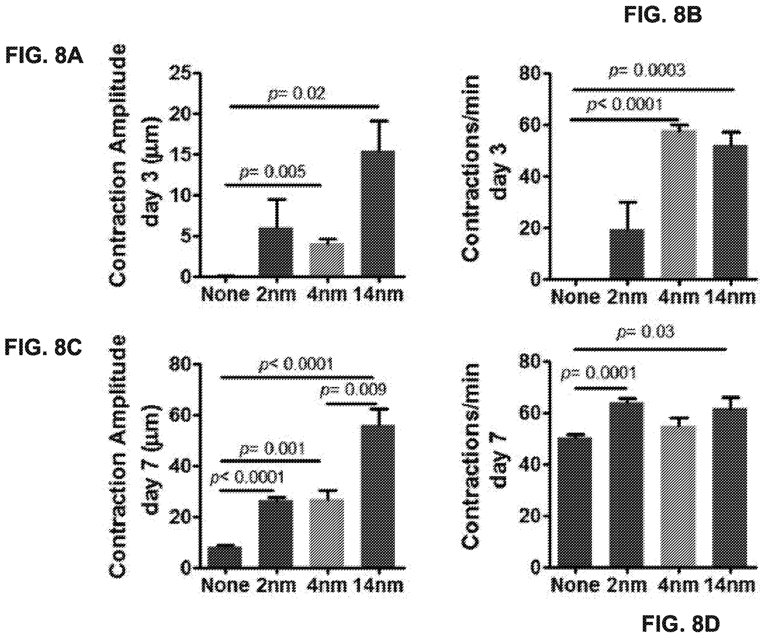

Finally, to evaluate the importance of gold NPs in scaffolds used for cardiac tissue engineering, the performances of the engineered tissues were evaluated. Since a strong contraction of an engineered tissue is essential for creating an effective heart patch³², contraction amplitudes of the cell constructs were evaluated by image analysis. While on day 3 no contraction was observed within tissues engineered without gold NPs, high contraction amplitudes were observed in all gold NP cell constructs (Figure 8). Tissues that were engineered within 4 and 14 nm-gold NP constructs revealed significantly higher amplitudes, compared to tissues engineered within pristine scaffolds (Figure 8A). Furthermore, on day 3 these engineered tissues exhibited significantly higher contraction rates, as compared to the ones engineered without gold NPs (Figure 8B). On day 7, contraction was observed within tissues engineered in the pristine scaffolds as well. However their contraction amplitude and rate were significantly lower than the gold NP tissues (Figures 8C, and 8D). The weak contraction force may be attributed to the rounded morphology of the cells and lack of tissue anisotropy. Recently it was shown that gold nanowires can transfer the electrical signal between adjacent cardiac cells ²⁰. This unique transfer phenomenon is important since post isolation and seeding cardiac cells lose their elongated morphology and acquire an immature rounded morphology²¹. At that point connexin 43 proteins, gap junction molecules responsible for transferring the electrical signal between cells, are internalized and the cells are required to establish new couplings. It is thought that the NPs in the present scaffolds were used to quickly promote cell-cell interactions, and thus improve the transfer of the electrical signal, and enhance the overall contractility in the engineered tissue.

### CONCLUSIONS

In this example, the incorporation of gold NPs onto the surface of fibrous scaffolds for engineering functional cardiac tissues is reported. The facile and inexpensive technique used for gold NP incorporation relied on immediate conjugation of the NPs to the functional groups of the protein comprising the fibers. Gold NP fiber scaffolds induced quick formation of elongated and aligned tissues with a morphology resembling that of cardiac cell bundles *in vivo.* Furthermore, it was shown that this structure has led to improved function, resulting in engineered tissues capable to generate a strong contraction force.

### REFERENCES FOR EXAMPLE 1

1 Dvir, T. et al. Prevascularization of cardiac patch on the omentum improves its therapeutic outcome. Proceedings of the National Academy of Sciences of the United States of America 106, 14990-14995, doi:10.1073/pnas.0812242106 (2009).
2 Zimmermann, W. H. et al. Engineered heart tissue grafts improve systolic and diastolic function in infarcted rat hearts. Nature medicine 12, 452-458, doi:10.1038/nm1394 (2006).
3 Godier-Furnemont, A. F. et al. Composite scaffold provides a cell delivery platform for cardiovascular repair. Proceedings of the National Academy of Sciences of the United States of America 108, 7974-7979, doi:10.1073/pnas.1104619108 (2011).
4 Sekine, H. et al. Endothelial cell coculture within tissue-engineered cardiomyocyte sheets enhances neovascularization and improves cardiac function of ischemic hearts. Circulation 118, S145-152, doi:10.1161/CIRCULATIONAHA.107.757286 (2008).
5 Fleischer, S. & Dvir, T. Tissue engineering on the nanoscale: lessons from the heart. Current opinion in biotechnology, doi:10.1016/j.copbio.2012.10.016 (2012).
6 Chiu, L. L., Janic, K. & Radisic, M. Engineering of oriented myocardium on three-dimensional micropatterned collagen-chitosan hydrogel. The International journal of artificial organs 35, 237-250, doi:10.5301/ijao.5000084 (2012).
7 Badie, N. & Bursac, N. Novel micropatterned cardiac cell cultures with realistic ventricular microstructure. Biophysical journal 96, 3873-3885, doi:10.1016/j.bpj.2009.02.019 (2009).
8 Heidi Au, H. T., Cui, B., Chu, Z. E., Veres, T. & Radisic, M. Cell culture chips for simultaneous application of topographical and electrical cues enhance phenotype of cardiomyocytes. Lab on a chip 9, 564-575, doi:10.1039/b810034a (2009).
9 Madden, L. R. et al. Proangiogenic scaffolds as functional templates for cardiac tissue engineering. Proceedings of the National Academy of Sciences of the United States of America 107, 15211-15216, doi:10.1073/pnas.1006442107 (2010).
10 Carrier, R. L. et al. Perfusion improves tissue architecture of engineered cardiac muscle. Tissue engineering 8, 175-188, doi:10.1089/107632702753724950 (2002).
11 Barash, Y. et al. Electric field stimulation integrated into perfusion bioreactor for cardiac tissue engineering. Tissue engineering. Part C, Methods 16, 1417-1426, doi:10.1089/ten.TEC.2010.0068 (2010).
12 Dvir, T., Benishti, N., Shachar, M. & Cohen, S. A novel perfusion bioreactor providing a homogenous milieu for tissue regeneration. Tissue engineering 12, 2843-2852, doi:10.1089/ten.2006.12.2843 (2006).
13 Radisic, M., Marsano, A., Maidhof, R., Wang, Y. & Vunjak-Novakovic, G. Cardiac tissue engineering using perfusion bioreactor systems. Nature protocols 3, 719-738, doi:10.1038/nprot.2008.40 (2008).
14 Shachar, M., Tsur-Gang, O., Dvir, T., Leor, J. & Cohen, S. The effect of immobilized RGD peptide in alginate scaffolds on cardiac tissue engineering. Acta biomaterialia 7, 152-162, doi:10.1016/j.actbio.2010.07.034 (2011).
15 Camelliti, P., McCulloch, A. D. & Kohl, P. Microstructured cocultures of cardiac myocytes and fibroblasts: a two-dimensional in vitro model of cardiac tissue. Microscopy and microanalysis : the official journal of Microscopy Society of America, Microbeam Analysis Society, Microscopical Society of Canada 11, 249-259, doi:10.1017/S 1431927605050506 (2005).
16 Kim, D. H. et al. Nanoscale cues regulate the structure and function of macroscopic cardiac tissue constructs. Proceedings of the National Academy of Sciences of the United States of America 107, 565-570, doi:10.1073/pnas.0906504107 (2010).
17 Kai, D., Prabhakaran, M. P., Jin, G. & Ramakrishna, S. Guided orientation of cardiomyocytes on electrospun aligned nanofibers for cardiac tissue engineering. Journal of biomedical materials research. Part B, Applied biomaterials 98B, 379-386, doi:10.1002/jbm.b.31862 (2011).
18 Bursac, N., Parker, K. K., Iravanian, S. & Tung, L. Cardiomyocyte cultures with controlled macroscopic anisotropy: a model for functional electrophysiological studies of cardiac muscle. Circulation research 91, e45-54 (2002).
19 Bien, H., Yin, L. & Entcheva, E. Cardiac cell networks on elastic microgrooved scaffolds. IEEE engineering in medicine and biology magazine : the quarterly magazine of the Engineering in Medicine & Biology Society 22, 108-112 (2003).
20 Dvir, T. et al. Nanowired three-dimensional cardiac patches. Nature nanotechnology 6, 720-725, doi:10.1038/nnano.2011.160 (2011).
21 Dvir, T., Timko, B. P., Kohane, D. S. & Langer, R. Nanotechnological strategies for engineering complex tissues. Nature nanotechnology 6, 13-22, doi:10.1038/nnano.2010.246 (2011).
22 Cohen-Karni, T. et al. Nanocomposite gold-silk nanofibers. Nano letters 12, 5403-5406, doi:10.1021/nl302810c (2012).
23 Homola, J. Surface plasmon resonance sensors for detection of chemical and biological species. Chem Rev 108, 462-493, doi:Doi 10.1021/Cr068107d (2008).
24 Maoz, B. M. et al. Amplification of Chiroptical Activity of Chiral Biomolecules by Surface Plasmons. Nano letters 13, 1203-1209, doi:Doi 10.1021/N1304638a (2013).
25 Khlebtsov, N. & Dykman, L. Biodistribution and toxicity of engineered gold nanoparticles: a review of in vitro and in vivo studies. Chem Soc Rev 40, 1647-1671, doi:Doi 10.1039/C0cs00018c (2011).
26 You, J. O., Rafat, M., Ye, G. J. & Auguste, D. T. Nanoengineering the heart: conductive scaffolds enhance connexin 43 expression. Nano letters 11, 3643-3648, doi:10.1021/nl201514a (2011).
27 Prabhakaran, M. P., Ghasemi-Mobarakeh, L. & Ramakrishna, S. Electrospun composite nanofibers for tissue regeneration. Journal of nanoscience and nanotechnology 11, 3039-3057 (2011).
28 Teo, W. E. & Ramakrishna, S. A review on electrospinning design and nanofibre assemblies. Nanotechnology 17, R89-R106, doi:10.1088/0957-4484/17/14/R01 (2006).
29 Orlova, Y., Magome, N., Liu, L., Chen, Y. & Agladze, K. Electrospun nanofibers as a tool for architecture control in engineered cardiac tissue. Biomaterials 32, 5615-5624, doi:10.1016/j.biomaterials.2011.04.042 (2011).
30 Tesler, A. B. et al. Tunable Localized Plasmon Transducers Prepared by Thermal Dewetting of Percolated Evaporated Gold Films. J Phys Chem C 115, 24642-24652, doi:Doi 10.1021/Jp209114j (2011).
31 Zhang, Y., Ouyang, H., Lim, C. T., Ramakrishna, S. & Huang, Z. M. Electrospinning of gelatin fibers and gelatin/PCL composite fibrous scaffolds. Journal of biomedical materials research. Part B, Applied biomaterials 72, 156-165, doi:10.1002/jbm.b.30128 (2005).
32 Iyer, R. K., Chiu, L. L., Reis, L. A. & Radisic, M. Engineered cardiac tissues. Current opinion in biotechnology 22, 706-714, doi:10.1016/j.copbio.2011.04.004 (2011).
33 Ma, Z., He, W., Yong, T. & Ramakrishna, S. Grafting of gelatin on electrospun poly(caprolactone) nanofibers to improve endothelial cell spreading and proliferation and to control cell Orientation. Tissue engineering 11, 1149-1158, doi:10.1089/ten.2005.11.1149 (2005).
34 Jaiswal, M., Koul, V., Dinda, A. K., Mohanty, S. & Jain, K. G. Cell adhesion and proliferation studies on semi-interpenetrating polymeric networks (semi-IPNs) of polyacrylamide and gelatin. Journal of biomedical materials research. Part B, Applied biomaterials 98B, 342-350, doi:10.1002/jbm.b.31857 (2011).
35 Mayer, K. M. & Hafner, J. H. Localized Surface Plasmon Resonance Sensors. Chem Rev 111, 3828-3857, doi:Doi 10.1021/Cr100313v (2011).
36 Engelmayr, G. C., Jr. et al. Accordion-like honeycombs for tissue engineering of cardiac anisotropy. Nature materials 7, 1003-1010, doi:10.1038/nmat2316 (2008).
37 Wheeldon, I., Farhadi, A., Bick, A. G., Jabbari, E. & Khademhosseini, A. Nanoscale tissue engineering: spatial control over cell-materials interactions. Nanotechnology 22, 212001, doi:10.1088/0957-4484/22/21/212001 (2011).
38 Lee, E. J., Vunjak-Novakovic, G., Wang, Y. & Niklason, L. E. A biocompatible endothelial cell delivery system for in vitro tissue engineering. Cell transplantation 18, 731-743, doi:10.3727/096368909X470919 (2009).
39 Marsano, A. et al. Engineering of functional contractile cardiac tissues cultured in a perfusion system. Conference proceedings : ... Annual International Conference of the IEEE Engineering in Medicine and Biology Society. IEEE Engineering in Medicine and Biology Society. Conference 2008, 3590-3593, doi:10.1109/IEMBS.2008.4649982 (2008).
40 Bellapadrona, G. et al. Optimization of localized surface plasmon resonance transducers for studying carbohydrate-protein interactions. Anal Chem 84, 232-240, doi:10.1021/ac202363t (2012).

### EXAMPLE 2

### PCL scaffolds coated with nanoparticles

### Materials and Methods

***Electrospinning:*** PCL (17.5% w/v, Mn 80,000, Sigma-Aldrich, St. Louis, MO) was dissolved in DCM and DMF in a ratio of 1:1. A syringe pump (Harvard apparatus, Holliston, MA) was used to deliver the polymer solution through a stainless steel 20G capillary at a rate of 0.5 mL/h. A high voltage power supply (Glassman high voltage Inc., Whitehouse Station, NJ) was used to apply a 17.5 kV potential between the capillary tip and the grounded aluminum collector placed 15 cm away. The obtained fibers were examined under a light microscope to verify coiled morphology and then air-dried for 48-72 h to allow residual solvent to evaporate.

***Gold NP preparation:*** Scaffolds, or single electrospun fibers were mounted in a VST e-beam evaporator. Au films (10 nm thick) were prepared by evaporation of Au (99.999%) from a tungsten boat at 1-3 × 10⁻⁶ torr at a deposition rate of 0.5 A s⁻¹.

***Topography and mechanical properties measurements by AFM:*** Topography and mechanical properties measurements were performed using a JPK research AFM (model NanoWizard III) in the force spectroscopy mode. NanoSensors, PPP-NCHR-50 scanning probe was used with a resonance frequency range of 204 - 497 kHz, force constant range of 10 - 130 N/m.

### Scanning electron microscopy:

***Coiled-fiber scaffolds.*** Scaffolds were mounted onto aluminum stubs with conductive paint and sputter-coated with an ultrathin (150 Å) layer of gold in a Polaron E 5100 coating apparatus. The samples were viewed under SEM (JEOL model JSM-840A) at an accelerating voltage of 25 kV.

***AuNPs coiled fibers.*** Fibers were imaged without additional coating using a Quanta 200 FEG Environmental Scanning Electron Microscope (ESEM) with a field-emission gun (FEG) electron source. Imaging was carried out under low vacuum with a high tension of 20 kV and a working distance of 7.3 mm.

***Cardiac cell isolation, seeding and cultivation:*** Cardiac cells were isolated as previously described.²⁶ Briefly, left ventricles of 0-3 day old neonatal Sprague-Dawley rats were harvested and cells were isolated using 6 cycles (30 min each) of enzyme digestion with collagenase type II (95 U/mL; Worthington, Lakewood, NJ) and pancreatin (0.6 mg/ml; Sigma-Aldrich) in Dulbecco's modified Eagle Medium (DMEM, (CaCl₂•2H₂0 (1.8 mM), KCl (5.36 mM), MgSO₄•7H₂O (0.81 mM), NaCl (0.1 M), NaHCO₃ (0.44 mM), NaH₂PO₄ (0.9 mM)). After each round of digestion cells were centrifuged (600 G, 5 min) and re-suspended in culture medium composed of M-199 (Biological Industries, Beit-Haemek, Israel) supplemented with 0.6 mM CuSO₄5•H₂O, 0.5 mM ZnSO₄•7H₂O, 1.5 mM vitamin B12, 500 U/mL Penicillin and 100 mg/ml streptomycin, and 0.5% (v/v) FBS. To enrich the cardiomyocytes population, cells were suspended in culture medium with 5% FBS and pre-plated twice (30 min). Following, cells were counted and seeded on the scaffolds using a single droplet. The cell-seeded constructs were cultivated at 37° C in a 5% carbon dioxide humidified incubator.

***Immunostaining:*** Immunostaining was performed as previously described.²⁷ Cardiac cell constructs were fixed and permeabilized in 100% cold methanol for 10 min, washed three times in DMEM-based buffer and then blocked for 1 h at room temperature in DMEM-based buffer containing 2% FBS. The samples were then incubated with primary antibodies to detect α-sarcomeric actinin (1:750, Sigma-Aldrich), washed three times, and incubated for 1 h with Alexa Fluor 647 conjugated goat anti-mouse antibody (1:500; Jackson, West Grove, PA) and Alexa Fluor 488 conjugated goat anti-rabbit antibody (1:500; Jackson). For nuclei detection, the cells were incubated for 3 min with Hoechst 33258 (1:100; Sigma-Aldrich) and washed three times. Samples were visualized using a confocal microscope (Nikon Eclipse Ni).

### Functional assessment:

***Contractions.*** Samples were filmed under a microscope (Nikon Eclipse TI, inverted) for functional assessments. Longitude change during tissue contraction was analyzed using image J (NIH). Contraction rate was counted. At least 5 samples from each group were used for analyses.

***Excitation threshold.*** Cell constructs were placed in Tyrode's solution at 37°C between two carbon electrode rods placed 1 cm apart in a Petri dish. Constructs were stimulated with 100 ms square pulses delivered at a rate of 2 Hz, starting with an amplitude of 1 V electrical field. The amplitude was increased by 0.1 V increments. Excitation threshold was defined as the voltage where the tissue construct started to contract synchronously at a frequency of 2 Hz.

***Statistical analysis:*** Statistical analysis data are presented as means ± SEM. Univariate differences between the pristine scaffold and AuNPs scaffolds were assessed with Student's t-test. All analyses were performed using GraphPad Prism version 5.00 for Windows (GraphPad Software), *p*< 0.05 was considered significant.

### RESULTS

Engineered cardiac patches require mechanical properties of the natural microenvironment. These properties are essential for efficient tissue contraction and relaxation. The natural heart matrix contains a unique subpopulation of coiled perimysial fibers.²¹ These fibers stretch and re-coil with the heart muscle, providing it with unique mechanical properties crucial for its efficient and continuous contractions.^{21,22}

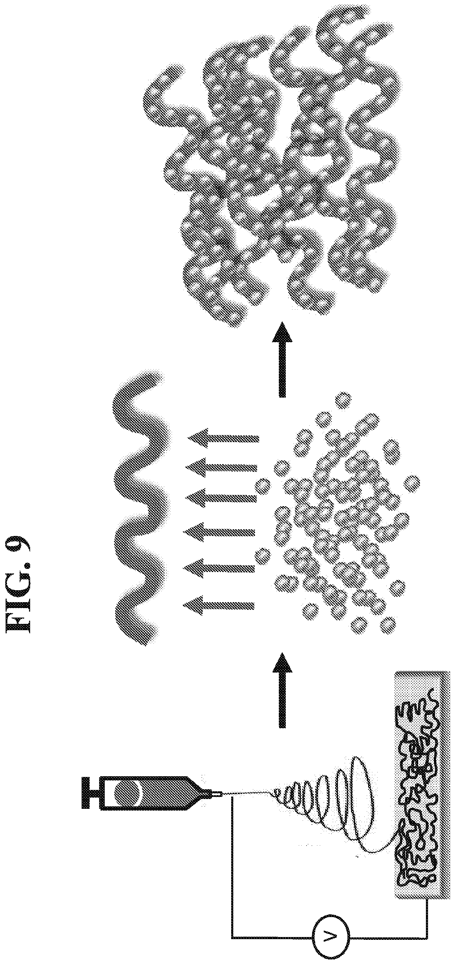

The present inventors sought to further improve cardiac patch performances on coiled fibers scaffolds by integration of gold nanoparticles (AuNPs) ensuring anisotropic transfer of the electrical signal throughout engineered cardiac tissues, with coiled fiber scaffolds. Figure 9 schematically illustrates the experimental process. Poly(ε-caprolactone) (PCL) was dissolved in a 1:1 ratio of dichloromethane (DCM) and dimethylformamide (DMF), and electrospun on a static collector to fabricate coiled fibers. The fibers were then placed in a VST e-beam evaporator and Au was deposited on their surface to create the nanocomposite coiled fiber scaffolds.

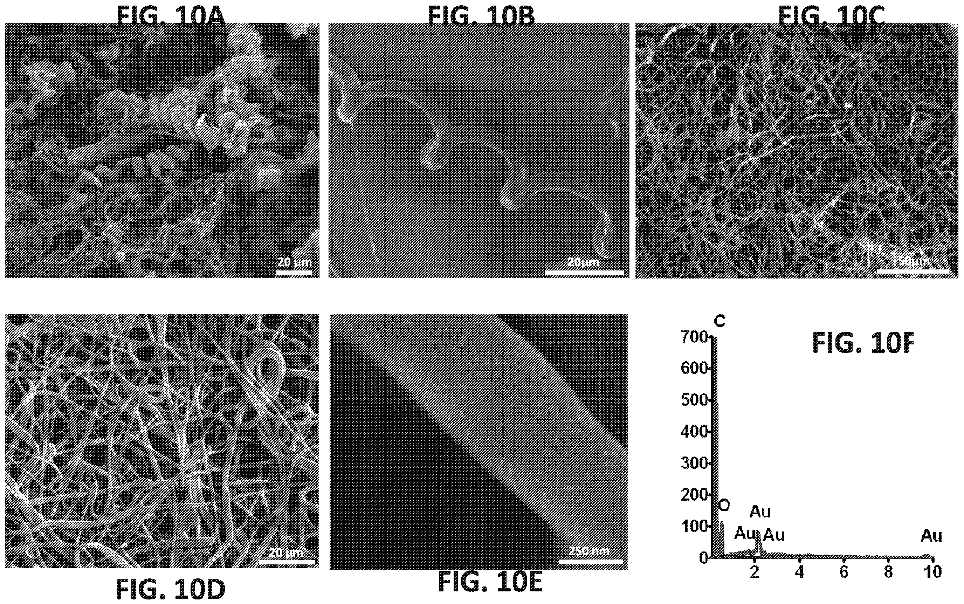

Inspired by the structure of natural coiled perimysial fibers (Figure 10A), the present inventors synthetically fabricated similar coiled electrospun fibers (Figure 10B). Scaffolds composed of such coiled fibers with diameters ranging between a few hundreds of nanometers to several micrometers exhibited an average pore area > 4000 µm² (Figures 10C,D).²³ They next sought to overcome the limited ability of the scaffolds to propagate the electrical signal between cultured cardiac cells by evaporating AuNPs with a nominal thickness of 10 nm onto the surface of the fibers. ESEM images revealed that AuNPs were homogenously distributed on the surface of the fibers (Figure 10E). Elemental mapping using energy dispersive x-ray spectroscopy (EDX) confirmed that the scaffolds were covered with AuNPs (Figure 10F).

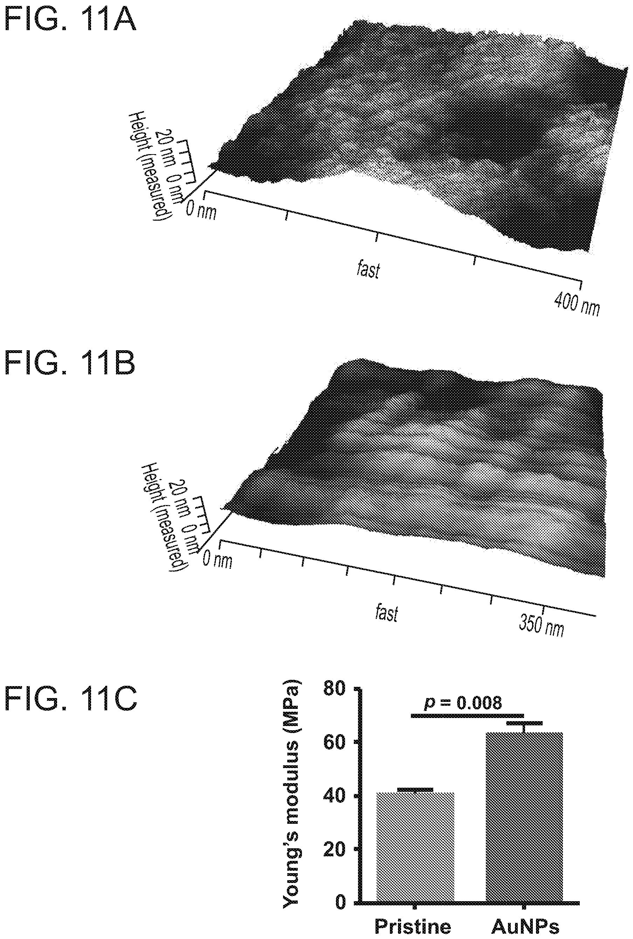

To investigate the effect of the AuNPs on the coiled fibers, single fibers were electrospun on slides and the z-axis-Young's modulus of fibers with and without AuNPs was investigated using atomic force microscope (AFM). Figures 11A and 11B revealed the topography of fibers with and without AuNPs. While the pristine fibers were relatively smooth, the AuNPs were clearly seen on the surface of the modified fibers. This allowed a relatively easy placement of the AFM tip on the AuNPs for measuring the mechanical properties of the composite fibers. As shown, the AuNPs significantly increased the elastic modulus of the fibers (Fig. *3B**; p=* 0.008).

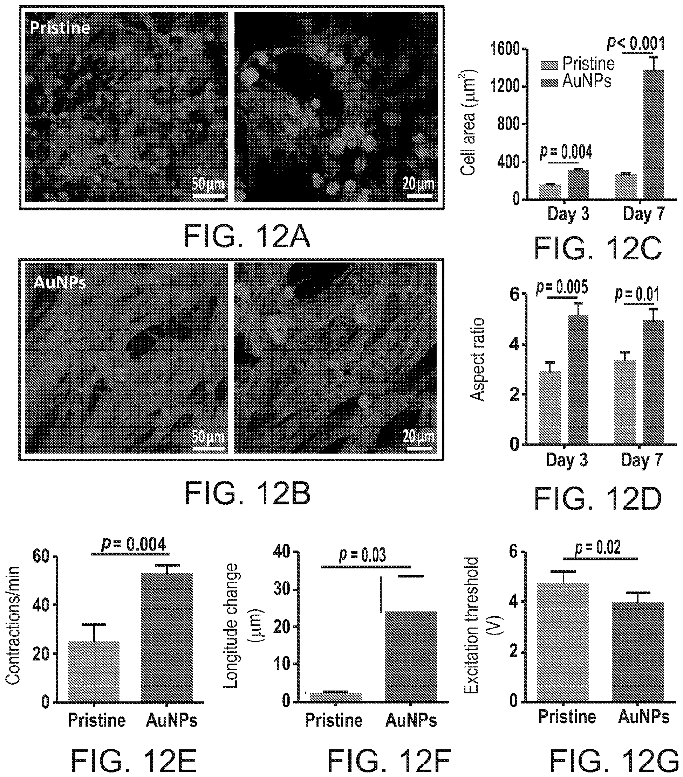

Next, the present inventors investigated the potential of the AuNPs-coiled fiber scaffolds (AuNPs scaffolds) to induce cardiac cell assembly into a mature tissue with morphological and biochemical hallmarks resembling those of the natural myocardium. Cardiac cells were isolated from neonatal rats and cultured for 7 days within pristine or AuNPs scaffolds. On day 7, engineered cardiac tissues were stained for α-sarcomeric actinin, a marker associated with cardiac muscle contraction. Figure 12A revealed that cardiac cells cultured within pristine scaffolds exhibited limited cell spreading and a rounded morphology. Furthermore, massive cell-cell interactions were not observed and cardiac cell bundles were not formed. In contrast, cardiac cells cultured within AuNPs scaffolds exhibited aligned and elongated morphology with massive actinin striation (Figure 12B). The cells organized into elongated and aligned cardiac-cell bundles, resembling the natural morphology of cell bundles in the myocardium.¹⁰ These results suggest the formation of a cardiac tissue with strong contraction potential. Analysis of cell area within the two types of scaffolds on days 3 and 7 revealed significantly larger cell area in the AuNPs scaffolds (Figure 12C; *p*=0.004 on day 3, and *p*<0.0001 on day 7 ). Moreover, analysis of cell elongation (presented as cell aspect ratio) revealed that on day 3 and 7 cardiac cells cultured within AuNPs scaffolds had significantly higher aspect ratio than those grown in pristine scaffolds (Fig. 4D; *p*=0.005 on day 3, and *p*=0.01 on day 7). Overall these results indicate that the AuNPs encouraged cardiac cell assembly into an organized and dense tissue with a strong and anisotropic contraction potential.

Finally, the present inventors assessed the potency of AuNPs scaffolds to induce the assembly of a functional cardiac tissue. In an attempt to improve heart function after MI an engineered cardiac patch should be able to generate a strong contraction force. ¹⁸ Analysis of tissue contraction rates revealed significantly higher rates in tissues cultured within the AuNPs scaffolds as compared to pristine scaffolds (Figure 12E). To evaluate the contraction force that the tissues can generate, the longitude change of the cardiac patches was measured on day 7. Cardiac tissues cultured within AuNPs scaffolds generated significantly stronger contraction forces as compared to the tissues grown within the pristine scaffolds (Figure 12F). Based on previous studies it was hypothesized that AuNPs will induce superior cell electrical coupling, leading to synchronous contraction of the entire tissue.^{11,20} To investigate this, cardiac constructs were subjected to external electrical field, increasing in increments of 0.1 V. Excitation threshold was defined as the minimum voltage needed to induce synchronous contractions of the entire patch at the defined frequency (higher than the normal contraction rate). Figure 12G revealed that cardiac tissues grown within AuNPs scaffolds reacted to significantly lower electrical fields compared to those grown in pristine scaffolds (*p*= 0.02). Overall, the results hinted at the superior function of cardiac tissues cultured within coiled fiber scaffolds incorporated with AuNPs.

### REFERENCES FOR EXAMPLE 2

1. A. S. Go, D. Mozaffarian, V. L. Roger, E. J. Benjamin, J. D. Berry, M. J. Blaha, et al., Heart Disease and Stroke Statistics--2014 Update: A Report From the American Heart Association, Circulation, 2013.
2. A. S. Go, D. Mozaffarian, V. L. Roger, E. J. Benjamin, J. D. Berry, W. B. Borden, et al., Executive summary: heart disease and stroke statistics--2013 update: a report from the American Heart Association, Circulation, 2013, 127(1), 143-152.
3. A. R. Williams, K. E. Hatzistergos, B. Addicott, F. McCall, D. Carvalho, V. Suncion, et al., Enhanced effect of combining human cardiac stem cells and bone marrow mesenchymal stem cells to reduce infarct size and to restore cardiac function after myocardial infarction, Circulation, 2013, 127(2), 213-223.
4. R. Bolli, A. R. Chugh, D. D'Amario, J. H. Loughran, M. F. Stoddard, S. Ikram, et al., Cardiac stem cells in patients with ischaemic cardiomyopathy (SCIPIO): initial results of a randomised phase 1 trial, Lancet, 2011, 378(9806), 1847-1857.
5. S. Fleischer and T. Dvir, Tissue engineering on the nanoscale: lessons from the heart, Current opinion in biotechnology, 2013, 24(4), 664-671.
6. J. Buikema, P. van der Meer, J. P. Sluijter and I. J. Domian, Engineering Myocardial Tissue: The Convergence of Stem Cells Biology and Tissue Engineering Technology, Stem Cells, 2013.
7. M. A. Laflamme and C. E. Murry, Heart regeneration, Nature, 2011, 473(7347), 326-335.
8. G. Vunjak-Novakovic, N. Tandon, A. Godier, R. Maidhof, A. Marsano, T. P. Martens, et al., Challenges in cardiac tissue engineering, Tissue engineering. Part B, Reviews, 2010, 16(2), 169-187.
9. T. Eschenhagen, A. Eder, I. Vollert and A. Hansen, Physiological aspects of cardiac tissue engineering, American journal of physiology. Heart and circulatory physiology, 2012, 303(2), H133-143.
10. T. Dvir, B. P. Timko, D. S. Kohane and R. Langer, Nanotechnological strategies for engineering complex tissues, Nature nanotechnology, 2011, 6(1), 13-22.
11. T. Dvir, B. P. Timko, M. D. Brigham, S. R. Naik, S. S. Karajanagi, O. Levy, et al., Nanowired three-dimensional cardiac patches, Nature nanotechnology, 2011, 6(11), 720-725.
12. M. Radisic, H. Park, H. Shing, T. Consi, F. J. Schoen, R. Langer, et al., Functional assembly of engineered myocardium by electrical stimulation of cardiac myocytes cultured on scaffolds, Proceedings of the National Academy of Sciences of the United States of America, 2004, 101(52), 18129-18134.
13. Y. Sapir, O. Kryukov and S. Cohen, Integration of multiple cell-matrix interactions into alginate scaffolds for promoting cardiac tissue regeneration, Biomaterials, 2011,32(7), 1838-1847.
14. B. Liau, N. Christoforou, K. W. Leong and N. Bursac, Pluripotent stem cell-derived cardiac tissue patch with advanced structure and function, Biomaterials, 2011, 32(35), 9180-9187.
15. G. C. Engelmayr, Jr., M. Cheng, C. J. Bettinger, J. T. Borenstein, R. Langer and L. E. Freed, Accordion-like honeycombs for tissue engineering of cardiac anisotropy, Nature materials, 2008, 7(12), 1003-1010.
16. T. Dvir, A. Kedem, E. Ruvinov, O. Levy, I. Freeman, N. Landa, et al., Prevascularization of cardiac patch on the omentum improves its therapeutic outcome, Proceedings of the National Academy of Sciences of the United States of America, 2009, 106(35), 14990-14995.
17. W. H. Zimmermann, I. Melnychenko, G. Wasmeier, M. Didie, H. Naito, U. Nixdorff, et al., Engineered heart tissue grafts improve systolic and diastolic function in infarcted rat hearts, Nature medicine, 2006, 12(4), 452-458.
18. R. K. Iyer, L. L. Chiu, L. A. Reis and M. Radisic, Engineered cardiac tissues, Current opinion in biotechnology, 2011, 22(5), 706-714.
19. N. Bursac, Y. Loo, K. Leong and L. Tung, Novel anisotropic engineered cardiac tissues: studies of electrical propagation, Biochemical and biophysical research communications, 2007, 361(4), 847-853.
20. M. Shevach, B. M. Maoz, R. Feiner, A. Shapira and T. Dvir, Nanoengineering gold particle composite fibers for cardiac tissue engineering, Journal of Materials Chemistry B, 2013, 1(39), 5210-5217.
21. T. F. Robinson, M. A. Geraci, E. H. Sonnenblick and S. M. Factor, Coiled perimysial fibers of papillary muscle in rat heart: morphology, distribution, and changes in configuration, Circulation research, 1988, 63(3), 577-592.
22. P. J. Hanley, A. A. Young, I. J. LeGrice, S. G. Edgar and D. S. Loiselle, 3-Dimensional configuration of perimysial collagen fibres in rat cardiac muscle at resting and extended sarcomere lengths, The Journal of physiology, 1999, 517 (Pt 3), 831-837.
23. S. Fleischer, R. Feiner, A. Shapira, J. Ji, X. Sui, H. Daniel Wagner, et al., Spring-like fibers for cardiac tissue engineering, Biomaterials, 2013, 34(34), 8599-8606.
24. T. Cohen-Karni, K. J. Jeong, J. H. Tsui, G. Reznor, M. Mustata, M. Wanunu, et al., Nanocomposite gold-silk nanofibers, Nano letters, 2012, 12(10), 5403-5406.
25. J. O. You, M. Rafat, G. J. Ye and D. T. Auguste, Nanoengineering the heart: conductive scaffolds enhance connexin 43 expression, Nano letters, 2011, 11(9), 3643-3648.
26. T. Dvir, N. Benishti, M. Shachar and S. Cohen, A novel perfusion bioreactor providing a homogenous milieu for tissue regeneration, Tissue engineering, 2006, 12(10), 2843-2852.
27. T. Dvir, O. Levy, M. Shachar, Y. Granot and S. Cohen, Activation of the ERK1/2 cascade via pulsatile interstitial fluid flow promotes cardiac tissue assembly, Tissue engineering, 2007, 13(9), 2185-2193.

### EXAMPLE 3

### Gold nanoparticle decellularized omentum scaffolds

***Decellularization of the omentum:*** Omenta of healthy six month old pigs were purchased from the institute of animal research in Kibutz Lahav, Israel. In order to separate the cells, the tissues were transferred to hypotonic buffer solution containing 10 mM Tris base, 5 mM ethylenediaminetetraacetic acid (EDTA) and 1 µM phenylmethylsulfonyl fluoride (PMSF) at pH 8.0 twice for one hour and then overnight. The following day, the tissues were rinsed in 70% ethanol for 30 min and twice in 100% ethanol (30 min each), then the polar lipids of the tissue were extracted by three 30 minutes washes of 100% acetone. In the next 24 hours the a-polar lipids were extracted by incubation in a 60:40 hexane:acetone solution (with 3 changes), followed by a 30 min 100% ethanol rinse. Subsequently, samples were placed in 70% ethanol overnight at 4°C. The next morning, after 10 min ethanol shake, the tissues were treated again with the hypotonic buffer of 10 mM Tris base and 5 mM EDTA for 2 hour (twice for 1 hour), followed by 16 hours in 0.5% SDS. The tissues were then washed thoroughly twice with PBS for 30 min and then were placed for 30 min in a solution of 50 mM Tris-lmM MgCl2 at pH 8.0. The tissues were then gently agitated in a nucleic degradation solution of 50 mM Tris, 1 mM MgCl2 0.1% BSA and 40 units/ml Benzonase® nuclease at pH 8.0 for 20 hours at 37°C. Finally the tissues were washed twice with 50 mM Tris at pH 8.0 (30 min each), once with 70% ethanol for 10-15 min and three times with sterile double distilled water before freezing overnight and then lyophilized.

***AuNP-omentum scaffold preparation:*** Decellularized omentum scaffolds were mounted in a VST e-beam evaporator. Au films (4 or 10 nm thick) were prepared by evaporation of Au (99.999%) from a tungsten boat at 1-3 × 10⁻⁶ torr at a deposition rate of 0.5 A s⁻¹.

***Scanning electron microscopy:*** Fibers were imaged without additional coating using a Quanta 200 FEG Environmental Scanning Electron Microscope (ESEM) with a field-emission gun (FEG) electron source. Imaging was carried out under high vacuum with tension of 20 kV and a working distance of 10 mm.

***Elemental composition:*** Elemental composition was performed using Oxford EDX and acquired using INCA Software.

***Current-Voltage diagrams (IV curves):*** Electrodes were evaporated on the omentum samples using a 0.9mm *4.5 mm shadow masks, with 0.5 mm gap. Evaporation was carried out using a VST-TFDS-680 thin film deposition system. Titanium (Ti) was used as the adhesion layer, thickness 10 nm, rate 0.5 A/sec. Top Gold (Au) layer of 100 nm thickness was evaporated at 0.5 A/sec. Evaporation was carried out under pressure of around 1.9E-7 torr. IV curves were obtained using 2636A Keithley sourcemeter, connected to a probe station.

***TEM:*** TEM measurements were performed on JEOL JEM-1200EX (Japan), operating at 80 kV. Images were captured using SIS Megaview III and iTEM the Tem imaging platform (Olympus).

***Cardiac cell isolation, seeding and cultivation:*** Cardiac cells were isolated as described herein above. Briefly, left ventricles of 0-3 day old neonatal Sprague-Dawley rats were harvested and cells were isolated using 6 cycles (30 min each) of enzyme digestion with collagenase type II (95 U/mL; Worthington, Lakewood, NJ) and pancreatin (0.6 mg/ml; Sigma-Aldrich) in Dulbecco's modified Eagle Medium (DMEM, (CaCl₂•2H₂O (1.8 mM), KCl (5.36 mM), MgSO₄•7H₂O (0.81 mM), NaCl (0.1 M), NaHCO₃ (0.44 mM), NaH₂PO₄ (0.9 mM)). After each round of digestion cells were centrifuged (600 G, 5 min) and re-suspended in culture medium composed of M-199 (Biological Industries, Beit-Haemek, Israel) supplemented with 0.6 mM CuSO₄5•H₂O, 0.5 mM ZnSO₄•7H₂O, 1.5 mM vitamin B12, 500 U/mL Penicillin and 100 mg/ml streptomycin, and 0.5% (v/v) FBS. To enrich the cardiomyocytes population, cells were suspended in culture medium with 5% FBS and pre-plated twice (30 min) to enrich cardiomyocyte population, pre plated cells from first pre-plating step were used for viability XTT assay. Following, cells were counted and seeded on the scaffolds using a single droplet. The cell-seeded constructs were cultivated at 37° C in a 5% carbon dioxide humidified incubator.

***Cell immunostaining:*** Cardiac cell constructs were fixed and permeabilized in 100% cold methanol for 10 min, blocked for 8 min at RT in Super Block (ScyTek laboratories, West Logan, UT). After 3 PBS washes, the samples were incubated with primary antibody to detect α-sarcomeric actinin (1:750, Sigma-Aldrich), washed three times, and incubated for 1 h with Alexa Fluor 647 conjugated goat anti-mouse antibody (1:500; Jackson, West Grove, PA). For nuclei detection, the cells were incubated for 3 min with Hoechst 33258 (1:100; Sigma-Aldrich) and washed three times. Samples were analyzed using a confocal microscope LSM 510 Meta (Zeiss, Germany).

***Assessment of cardiac fibroblasts viability:*** Cardiac fibroblasts isolated in the first pre-plating stage of cell isolation process were subjected to 3 day expansion and after seeded in the scaffolds. Cardiac fibroblasts was determined by a colorimetric XTT assay for the quantification of cell proliferation and viability (Biological Industries) according to the manufacturer's instructions. The absorbance of each sample (475 nm) was measured against a background control (665 nm) using a SynergyHT micro-plate photometer.

***Functional assessment:** Contractions.* Samples were filmed under a microscope (Nikon Eclipse TI, inverted) for functional assessments. Longitude change during tissue contraction was analyzed using image J (NIH). At least 5 samples from each group were used for analyses. *Excitation threshold.* Cell constructs were placed in Tyrode's solution at 37°C between two carbon electrode rods placed 1 cm apart in a Petri dish. Constructs were stimulated with 100 ms square pulses delivered at a rate of 2 Hz, starting with an amplitude of 1 V electrical field. The amplitude was increased by 0.1 V increments. Excitation threshold was defined as the voltage where the tissue construct started to contract synchronously at a frequency of 2 Hz.

*Calcium transient measurements.* Neonatal rat ventricular myocytes were incubated with 10mM fluo-4 AM (Invitrogen) and 0.1% Pluronic F-127 for 45 min at 37°C. Cardiac cell constructs were subsequently washed three times in modified Tyrode solution to allow de-esterification. The calcium transients were imaged using Nikon eclipse Ti inverted research microscope. The images were acquired with a Hamamatsu ORCA - flash 4.0 camera at 100 frame/s with 3 µm/pixel spatial resolution Intensity of fluorescence over time was measured using ImageJ.

***VEGF immunostaining:*** Omentum scaffolds with or without 10 nm AuNP were incubated for 2 hr with VEGF (PeproTech Asia, 0.5 ug/ml), washed with PBS buffer and transferred into a humidified cell incubator. The next day scaffolds were stained with primary mouse monoclonal anti VEGF (1:100; Abcam) antibody and counter stained with Alexa Fluor 647 conjugated goat anti-mouse antibody (1:500; Jackson, West Grove, PA).

***Statistical analysis:*** Statistical analysis data are presented as means ± SEM. Univariate differences between the pristine scaffold and AuNPs scaffolds were assessed with Student's t-test. All analyses were performed using GraphPad Prism version 5.00 for Windows (GraphPad Software), *p* < 0.05 was considered significant.

### RESULTS

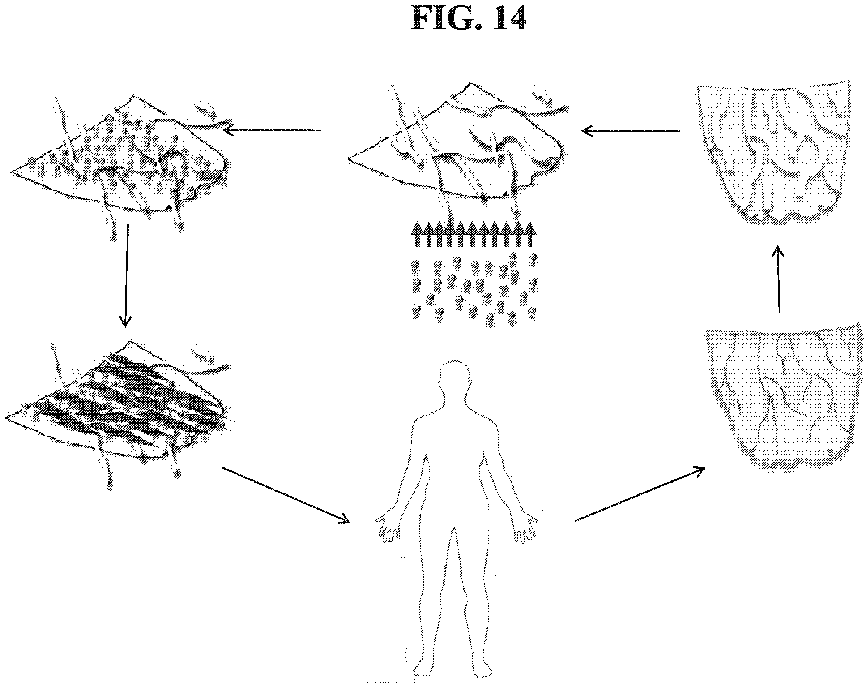

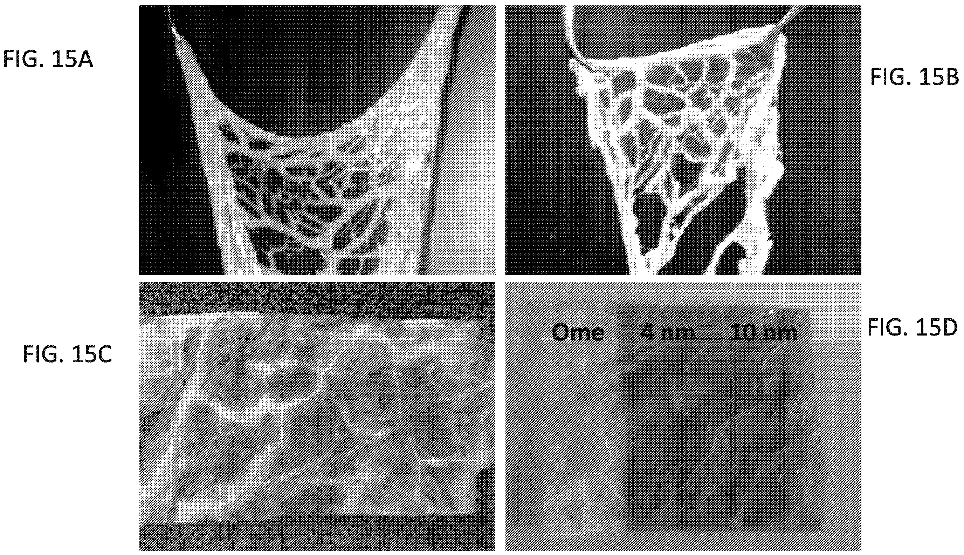

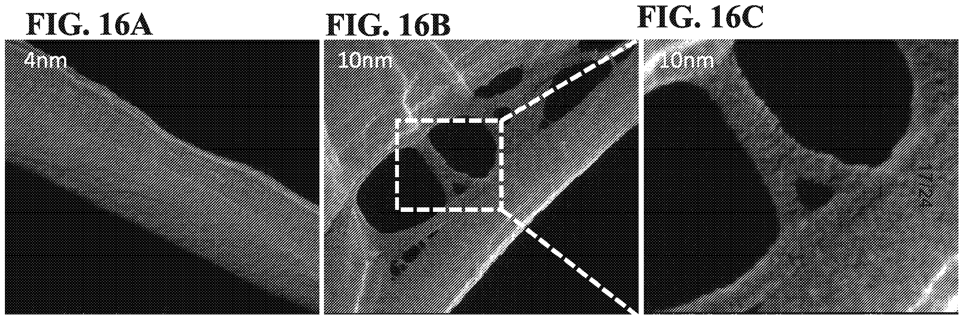

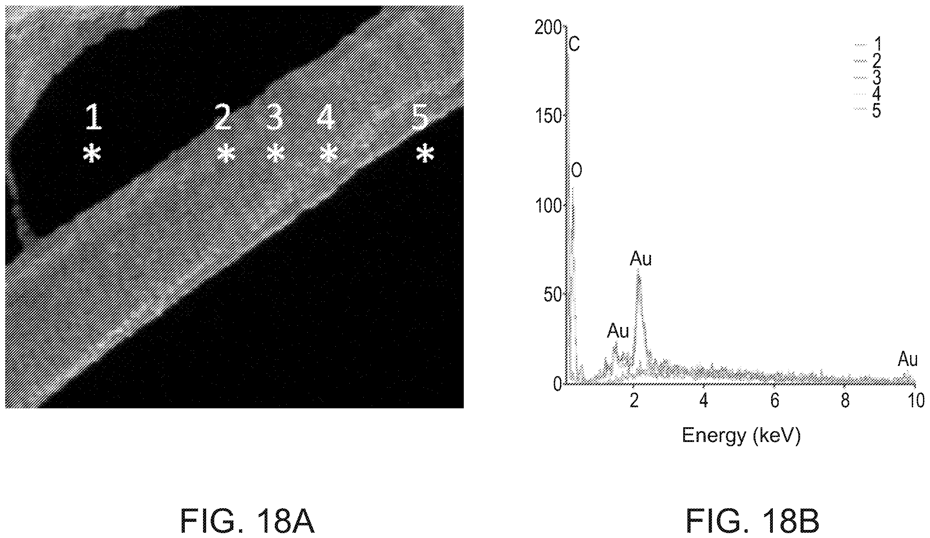

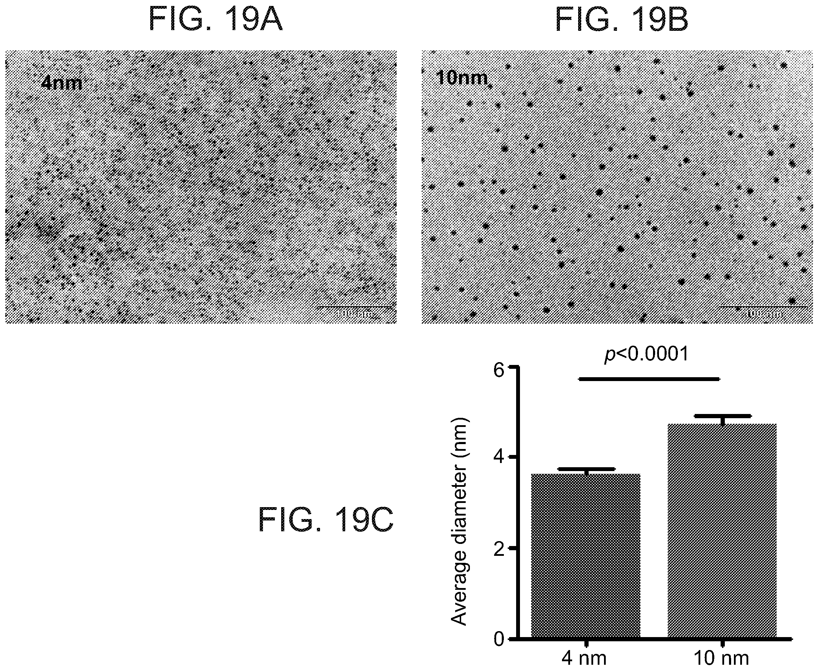

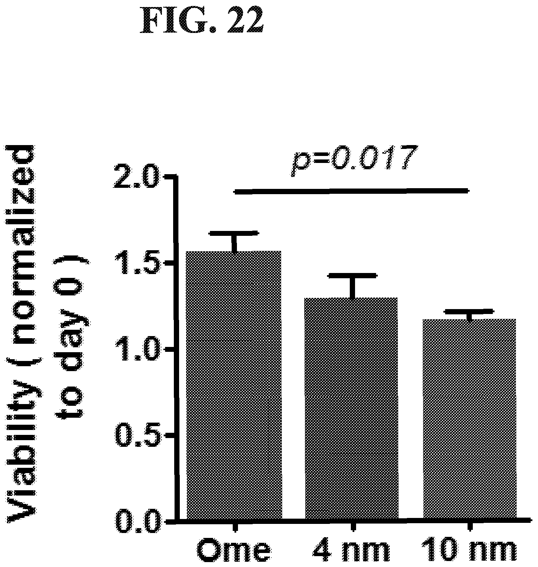

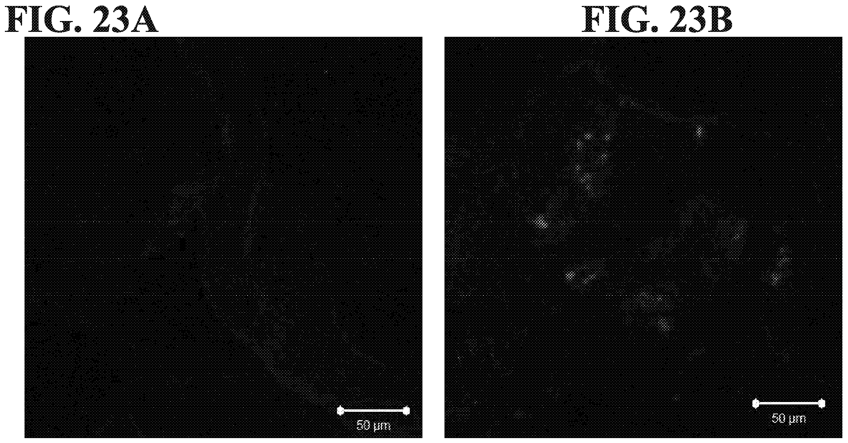

The results are illustrated in Figures 14-23.

The work leading to this invention has received funding from the People Programme (Marie Curie Actions) of the European Union's Seventh Framework Programme (EP72007-2013) under the REA grant agreement No. 303710.

## Claims

1. A composition of matter comprising viable electrically excitable cells seeded on a scaffold, wherein an outer surface of said scaffold comprises a coating of metal nanoparticles and wherein more than 50 % of said metal nanoparticles are positioned on said outer surface of said scaffold, wherein said coating is between 2-20 nm in thickness.

2. The composition of matter of claim 1, wherein said metal nanoparticles comprise gold nanoparticles.

3. The composition of matter of claim 1, wherein said scaffold comprises fibers.

4. The composition of matter of any one of claims 1-2, wherein said coating is between 4-14 nm in thickness.

5. The composition of matter of claim 1, wherein said scaffold comprises decellularized extracellular matrix (ECM).

6. The composition of matter of claim 1, wherein said metal nanoparticles are non-homogeneously distributed in said coating.

7. The composition of matter of claim 1, wherein said metal nanoparticles are attached to an antibody.

8. A method of generating a scaffold seeded with cells comprising:
(a) evaporating metal nanoparticles on the outer surface of the scaffold to generate a coating being between 2-20 nm in thickness; and subsequently
(b) seeding electrically excitable cells on said scaffold thereby generating the scaffold seeded with cells.

9. The method of claim 8, wherein said metal comprises gold.

10. The method of claim 8, further comprising electrospinning fibers of the scaffold prior to step (a).

11. The method of claim 8, further comprising decellularizing a tissue to generate fibers of the scaffold prior to step (a).

12. The method of claim 8, wherein said coating is between 4-14 nm in thickness.

13. The composition of matter of any one of claims 1-7, for use in treating a disease or disorder associated with a decrease in activity or amount of electrically excitable cells.

## Patentansprüche

1. Materialzusammensetzung, die vitale elektrisch erregbare Zellen umfasst, die auf einem Gerüst platziert sind, wobei eine äußere Oberfläche des Gerüsts eine Beschichtung aus Metallnanopartikeln umfasst und wobei mehr als 50 % der Metallnanopartikel auf der äußeren Oberfläche des Gerüsts angeordnet sind, wobei die Beschichtung zwischen 2 bis 20 nm dick ist.

2. Materialzusammensetzung nach Anspruch 1, wobei die Metallnanopartikel Goldnanopartikel umfassen.

3. Materialzusammensetzung nach Anspruch 1, wobei das Gerüst Fasern umfasst.

4. Materialzusammensetzung nach einem der Ansprüche 1-2, wobei die Beschichtung eine Dicke zwischen 4-14 nm aufweist.

5. Materialzusammensetzung nach Anspruch 1, wobei das Gerüst eine dezellularisierte extrazelluläre Matrix (ECM) umfasst.

6. Materialzusammensetzung nach Anspruch 1, wobei die Metallnanopartikel in der Beschichtung nicht-homogen verteilt sind.

7. Materialzusammensetzung nach Anspruch 1, wobei die Metallnanopartikel an einem Antikörper befestigt sind.

8. Verfahren zur Erzeugung eines Gerüstes, auf dem Zellen platziert sind, umfassend
(a) Verdampfen von Metallnanopartikeln auf der äußeren Oberfläche des Gerüsts, um eine Beschichtung mit einer Dicke zwischen 2-20 nm zu erzeugen; und anschließend
(b) Platzieren von elektrisch erregbaren Zellen auf dem Gerüst, wodurch das Gerüst, auf dem die Zellen platziert sind, erzeugt wird.

9. Verfahren nach Anspruch 8, wobei das Metall Gold umfasst.

10. Verfahren nach Anspruch 8, des Weiteren umfassend Elektrospinnen von Fasern des Gerüsts vor dem Schritt (a).

11. Verfahren nach Anspruch 8, des Weiteren umfassend Dezellularisieren von Gewebe, um Fasern des Gerüsts zu erzeugen, vor dem Schritt (a).

12. Das Verfahren nach Anspruch 8, wobei die Beschichtung eine Dicke zwischen 4-14 nm aufweist.

13. Materialzusammensetzung nach einem der Ansprüche 1-7, zur Verwendung bei der Behandlung einer Krankheit oder Störung, die mit einer Abnahme der Aktivität oder der Menge an elektrisch erregbaren Zellen verbunden ist.

## Revendications

1. Composition de matière comprenant des cellules viables électriquement excitables ensemencées sur un échafaudage, dans laquelle une surface extérieure dudit échafaudage comprend un revêtement de nanoparticules métalliques et dans laquelle plus de 50 % desdites nanoparticules métalliques sont positionnées sur ladite surface extérieure dudit échafaudage, dans laquelle ledit revêtement a une épaisseur comprise entre 2 et 20 nm.

2. Composition de matière selon la revendication 1, dans laquelle lesdites nanoparticules métalliques comprennent des nanoparticules d'or.

3. Composition de matière selon la revendication 1, dans laquelle ledit échafaudage comprend des fibres.

4. Composition de matière selon l'une quelconque des revendications 1 à 2, dans laquelle ledit revêtement a une épaisseur comprise entre 4 et 14 nm.

5. Composition de matière selon la revendication 1, dans laquelle ledit échafaudage comprend une matrice extracellulaire décellularisée (ECM).

6. Composition de matière selon la revendication 1, dans laquelle lesdites nanoparticules métalliques ne sont pas réparties de manière homogène dans ledit revêtement.

7. Composition de matière selon la revendication 1, dans laquelle lesdites nanoparticules métalliques sont liées à un anticorps.

8. Procédé pour générer un échafaudage ensemencé de cellules comprenant :
(a) une évaporation de nanoparticules métalliques sur la surface extérieure de l'échafaudage pour générer un revêtement ayant une épaisseur comprise entre 2 et 20 nm ; et ensuite
(b) un ensemencement de cellules électriquement excitables sur ledit échafaudage de façon à générer l'échafaudage ensemencé de cellules.

9. Procédé selon la revendication 8, dans lequel ledit métal comprend de l'or.

10. Procédé selon la revendication 8, comprenant en outre un électrofilage de fibres de l'échafaudage préalablement à l'étape (a).

11. Procédé selon la revendication 8, comprenant en outre la décellularisation d'un tissu pour générer des fibres de l'échafaudage préalablement à l'étape (a).

12. Procédé selon la revendication 8, dans lequel ledit revêtement a une épaisseur comprise entre 4 et 14 nm.

13. Composition de matière selon l'une quelconque des revendications 1 à 7, pour une utilisation dans le traitement d'une maladie ou d'un trouble associé à une diminution de l'activité ou de la quantité de cellules électriquement excitables.
